(19) Europäisches Patentamt
European Patent Office
Office européen des brevets

(11) **EP 3 236 241 B1**

# (12) FASCICULE DE BREVET EUROPEEN

(45) Date de publication et mention
de la délivrance du brevet:
**14.12.2022 Bulletin 2022/50**

(21) Numéro de dépôt: **17167013.6**

(22) Date de dépôt: **19.04.2017**

(51) Classification Internationale des Brevets (IPC):
**G01N 21/47** (2006.01)      **G01N 33/483** (2006.01)

(52) Classification Coopérative des Brevets (CPC):
**G01N 21/4795; G01N 33/4833;** G01N 21/4738;
G01N 2021/4709; G01N 2021/4773

(54) **PROCÉDÉ ET DISPOSITIF D'ESTIMATION DE PROPRIÉTÉS OPTIQUES D'UN ÉCHANTILLON**

VERFAHREN UND VORRICHTUNG ZUM ABSCHÄTZEN OPTISCHER EIGENSCHAFTEN EINER PROBE

METHOD AND DEVICE FOR MEASURING OPTICAL CHARACTERISTICS OF A SAMPLE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **22.04.2016 FR 1653587**

(43) Date de publication de la demande:
**25.10.2017 Bulletin 2017/43**

(73) Titulaires:
- **COMMISSARIAT À L'ÉNERGIE ATOMIQUE ET AUX
ÉNERGIES ALTERNATIVES**
**75015 Paris (FR)**
- **CENTRE NATIONAL DE LA RECHERCHE SCIENTIFIQUE**
**75016 Paris (FR)**
- **Université Pierre et Marie Curie**
**75252 Paris Cedex 05 (FR)**

(72) Inventeurs:
- **SORGATO, Véronica**
**QUITO (EC)**
- **BERGER, Michel**
**38640 CLAIX (FR)**
- **PLANAT-CHRETIEN, Anne**
**38120 ST EGREVE (FR)**
- **VEVER-BIZET, Christine**
**75008 PARIS (FR)**
- **BOURG-HECKLY, Geneviève**
**75003 PARIS (FR)**
- **EMAIN, Charlotte**
**38140 IZEAUX (FR)**

(74) Mandataire: **INNOV-GROUP**
**310, avenue Berthelot**
**69372 Lyon Cedex 08 (FR)**

(56) Documents cités:
**EP-A2- 2 762 064     WO-A2-2006/086085**

- **Brandon S Nichols ET AL: "A Quantitative Diffuse Reflectance Imaging (QDRI) System for Comprehensive Surveillance of the Morphological Landscape in Breast Tumor Margins", PloS one, 1 janvier 2015 (2015-01-01), page e0127525, XP055337212, United States DOI: 10.1371/journal.pone.0127525 Extrait de l'Internet: URL:http://journals.plos.org/plosone/artic le/file?id=10.1371/journal.pone.0127525&ty pe=printable [extrait le 2017-01-20]**
- **SULOCHANA DHAR ET AL: "A diffuse reflectance spectral imaging system for tumor margin assessment using custom annular photodiode arrays", BIOMEDICAL OPTICS EXPRESS, vol. 3, no. 12, 8 novembre 2012 (2012-11-08), pages 3211-3222, XP055337206,**
- **Brandon S Nichols ET AL: "Performance of a lookup table-based approach for measuring tissue optical properties with diffuse optical spectroscopy", Journal of Biomedical Optics, 20 avril 2012 (2012-04-20), page 057001, XP055337216, United States DOI: 10.1117/1.JBO.17.5.057001 Extrait de l'Internet: URL:http://biomedicaloptics.spiedigitallib rary.org/pdfaccess.ashx?resourceid=3469718 &pdfsource=24 [extrait le 2017-01-23]**

**(Cont. page suivante)**

• BJORGAN ASGEIR ET AL: "Estimation of skin optical parameters for real-time hyperspectral imaging applications", INTERNATIONAL SOCIETY FOR OPTICAL ENGINEERING, SPIE, PO BOX 10 BELLINGHAM WA 98227-0010 USA, vol. 19, no. 6, 1 juin 2014 (2014-06-01), page 66003, XP060047425, ISSN: 1083-3668, DOI: 10.1117/1.JBO.19.6.066003 [extrait le 2014-06-04]

**Description**

## DOMAINE TECHNIQUE

L'invention se situe dans le domaine de la caractérisation de propriétés optiques d'un échantillon, par exemple un échantillon de peau.

## ART ANTERIEUR

Les mesures optiques, permettant de caractériser des propriétés optiques d'échantillons, sont répandues. On peut notamment citer des mesures basées sur la détection d'un signal rétrodiffusé ou réfléchi par un échantillon illuminé par un faisceau lumineux. Il peut par exemple s'agir de la spectrométrie Raman, de l'imagerie de fluorescence ou de la spectrométrie de réflectance diffuse.

La spectrométrie de réflectance diffuse, usuellement désignée par l'acronyme anglosaxon DRS (Diffuse Reflectance Spectroscopy), consiste à exploiter la lumière rétrodiffusée par un objet diffusant soumis à une illumination ponctuelle. Cette technique a été décrite dans le document EP2762064 ainsi que dans la publication Sorgato V. "Non-contact quantitative diffuse reflectance spectroscopy", Proceedings of SPIE Vol. 9538, 95380U (2015), désignée par la suite par « Sorgato 2015 ». Ces documents décrivent une sonde comportant une fibre optique centrale, reliée à une source de lumière et destinée à diriger un faisceau lumineux fin vers un échantillon, en particulier un échantillon de peau. Des fibres optiques disposées autour de la fibre optique centrale, dites fibres de détection, recueillent un rayonnement optique rétrodiffusé par l'échantillon. Des moyens d'analyse spectrale du rayonnement rétrodiffusé permettent d'estimer simultanément des propriétés optiques de l'échantillon, en particulier des propriétés optiques de diffusion et des propriétés optiques d'absorption. La sonde peut être appliquée au contact ou à distance de l'échantillon à caractériser. Cette technique permet une caractérisation quantitative de ces propriétés optiques, et cela de façon simultanée, mais il s'agit d'une caractérisation locale. Les propriétés optiques sont déterminées dans un volume élémentaire de l'échantillon, ce volume élémentaire dépendant de la géométrie de la sonde. Les mesures par DRS peuvent mettre en oeuvre des modélisations du transport de photons dans l'échantillon observé, comme décrit par exemple dans la publication Nichols B "Performance of a lookup table-based approach for measuring tissue optical properties with diffuse optical spectroscopy" Journal of Biomedical Optics, Vol 17(5), May 2012.

Il est possible d'effectuer des mesures de type DRS en différentes positions discrètes sur un échantillon, selon un maillage spatial, de façon à obtenir une caractérisation bidimensionnelle des propriétés optiques de l'échantillon. Une telle démarche est présentée dans la publication Nichols BS et al. "A Quantitative Diffuse Reflectance Imaging (QDRI) System for Comprehensive Surveillance of the Morphological Landscape in Breast Tumor Margins", PLoS ONE 10, 2015, désignée par la suite par "Nichols 2015". Le dispositif et la méthode décrits dans cette publication permettent une caractérisation des propriétés optiques selon un maillage bidimensionnel discret comprenant 49 points répartis régulièrement selon un arrangement matriciel.

La publication Dhar S. "A diffuse reflectance spectral imaging system for tumor imaging assessment using custom annular photodiode arrays", Biomedical Optics Express, vol.3 n° 12, 2012 décrit également un système de mesure par DRS agencé selon une matrice, chaque point de la matrice comportant un photodétecteur annulaire. Une telle géométrie permet d'augmenter la quantité de rayonnement rétrodiffusé détectée. Cependant, un photodétecteur annulaire ne permet de quantifier le rayonnement rétrodiffusé par un échantillon n'émanant de ce dernier qu'à une seule distance autour d'un point d'illumination.

De façon alternative, à la DRS, l'imagerie spectrale de réflectance, désignée par l'acronyme anglosaxon MSI « Multi spectral Imaging » permet une caractérisation bidimensionnelle d'une propriété optique d'un échantillon. La publication Bjorgan A, "Estimation of skin optical parameters for real-time hyperspectral imaging applications", J. Biomed. Opt. 19(6), 066003, Jun 04, 2014, désignée par la suite par « Bjorgan 2014 », en est un exemple. Cette publication décrit l'utilisation d'images d'un échantillon, formées dans différentes bandes spectrales, pour évaluer une distribution spatiale bidimensionnelle du coefficient d'absorption de l'échantillon, et cela dans les différentes bandes spectrales. L'évaluation se base sur une valeur du coefficient d'absorption réduit préalablement déterminé en se basant sur des données disponibles dans la littérature. Étant basée sur des images, cette méthode peut être appliquée selon un large champ d'observation, et présente à la fois des bonnes résolutions spatiale et spectrale. Cependant, comme décrit par la suite, les inventeurs ont constaté qu'une telle méthode peut aboutir à une erreur de mesure importante lorsqu'on cherche à obtenir une quantification précise du coefficient d'absorption.

Le document WO 2006/086085 A2 décrit un procédé selon le préambule de la revendication 1.

Les inventeurs ont recherché une méthode permettant une caractérisation quantitative des propriétés optiques de diffusion et d'absorption d'un échantillon, conférant un champ d'observation étendu, de façon à obtenir rapidement une distribution spatiale bidimensionnelle de ces propriétés optiques. Cette méthode met en œuvre des dispositifs simples et peu coûteux, et est suffisamment robuste pour pouvoir être appliquée au-delà d'un laboratoire de recherche, par exemple dans une salle d'opération ou dans un laboratoire d'analyses.

EXPOSE DE L'INVENTION

Un objet de l'invention est un procédé d'estimation de propriétés optiques d'un échantillon, comme défini dans la revendication 1.

Ainsi, la propriété optique auxiliaire, mesurée ponctuellement sur l'échantillon, est utilisée pour déterminer la première propriété optique dudit échantillon.

Le premier faisceau lumineux est émis, par la première source de lumière, dans une première bande spectrale. De préférence, la première propriété optique et la propriété optique auxiliaire sont déterminées dans une même bande spectrale.

Selon un mode de réalisation, la première propriété optique est une propriété d'absorption de la lumière de l'échantillon, la propriété optique auxiliaire étant une propriété de diffusion de la lumière dans l'échantillon. Selon un autre mode de réalisation, la première propriété optique est une propriété de diffusion de la lumière de l'échantillon, la propriété optique auxiliaire étant une propriété d'absorption de la lumière dans l'échantillon.

De préférence, l'étape de mesure de la propriété optique auxiliaire comporte les sous-étapes suivantes :

i) illumination de la surface de l'échantillon à l'aide d'un faisceau lumineux auxiliaire, produit par une source de lumière auxiliaire, de façon à former, à la surface de l'échantillon, une zone élémentaire d'illumination, correspondant à la partie de la surface illuminée par ledit faisceau lumineux auxiliaire ;

ii) mesure, à l'aide d'un photodétecteur auxiliaire, d'un signal de rétrodiffusion, représentatif d'un rayonnement rétrodiffusé par l'échantillon, ledit rayonnement rétrodiffusé émanant d'une zone élémentaire de rétrodiffusion, séparée de la zone élémentaire d'illumination et située à une distance, dite distance de rétrodiffusion, de cette dernière ;

iii) estimation de la propriété optique auxiliaire de l'échantillon en fonction du signal de rétrodiffusion mesuré lors de la sous-étape ii).

Le faisceau lumineux auxiliaire est émis, par la source de lumière auxiliaire, dans une deuxième bande spectrale. La première bande spectrale et la deuxième bande spectrale se recouvrent ou sont confondues.

La sous-étape ii) peut comporter l'obtention, à partir du signal de rétrodiffusion, d'une grandeur d'intérêt dite auxiliaire, représentative d'une quantité du rayonnement rétrodiffusé par l'échantillon, à une distance de rétrodiffusion, suite à son illumination par le faisceau lumineux auxiliaire; la sous-étape iii) comporte alors une comparaison de ladite grandeur d'intérêt auxiliaire avec une pluralité d'estimations de cette grandeur d'intérêt auxiliaire, chaque estimation étant réalisée en considérant une valeur prédéterminée de ladite propriété optique auxiliaire. La propriété optique auxiliaire est alors obtenue en déterminant la propriété optique auxiliaire minimisant ladite comparaison.

De préférence, la sous-étape ii) est effectuée en considérant différentes distances de rétrodiffusion, l'étape iii) prenant en compte les signaux de rétrodiffusion respectivement mesurés à chacune de ces distances de rétrodiffusion.

Selon un mode de réalisation, lors de la sous-étape i), l'échantillon est illuminé successivement ou simultanément par une pluralité de faisceaux lumineux auxiliaires de façon à former, à la surface de ce dernier, une pluralité de zones élémentaires d'illumination espacées les unes des autres ; les sous-étapes ii) et iii) sont alors mises en œuvre en considérant un signal rétrodiffusé émis à au moins une distance de rétrodiffusion de chaque zone élémentaire d'illumination, de façon à estimer la propriété optique auxiliaire dans autant d'éléments de surface que de faisceaux lumineux auxiliaires

Selon un mode de réalisation, l'étape d) comporte une comparaison de la première grandeur d'intérêt déterminée lors de l'étape c) avec une pluralité d'estimations de cette grandeur d'intérêt, chaque estimation étant réalisée en considérant une valeur prédéterminée de la première propriété optique, et en prenant en compte la propriété optique auxiliaire préalablement mesurée sur l'échantillon. La première propriété optique peut notamment être obtenue en déterminant la propriété optique minimisant ladite comparaison.

Selon un mode de réalisation, l'étape c) comporte une segmentation de la première image en différentes régions d'intérêts, une première grandeur d'intérêt étant déterminée en chaque région d'intérêt. La segmentation peut être réalisée en fonction de l'intensité de pixels de l'image, ou en se basant sur une estimation a priori de la composition de l'échantillon au niveau de chaque région d'intérêt. Chaque région d'intérêt de l'image est optiquement couplée à une région d'intérêt de la surface de l'échantillon. Une région d'intérêt de l'image peut comprendre un pixel ou une pluralité de pixels de ladite image. L'étape c) peut comprendre les sous-étapes suivantes :

i) détermination d'une intensité représentative de chaque région d'intérêt de la première image ;
ii) application d'un premier facteur de calibration à ladite intensité représentative de chaque région d'intérêt, de façon à obtenir ladite première grandeur d'intérêt.

Le premier facteur de calibration peut être obtenu à partir d'un échantillon de calibration dont les propriétés optiques sont connues. Il peut représenter un ratio entre une quantité de rayonnement rétrodiffusé par ledit échantillon de calibration, cette quantité étant modélisée, sur une intensité mesurée en plaçant ledit échantillon de calibration à la place de l'échantillon examiné.

Selon un mode de réalisation, les étapes c) à d) sont mises en œuvre de façon itérative en considérant, lors de la première itération, un premier facteur de calibration correspondant à un échantillon de calibration choisi arbitrairement. Au cours d'une itération suivante, lors de l'étape c), la première grandeur d'intérêt est déterminée en appliquant un premier facteur de calibration correspondant à la première propriété optique estimée lors de l'étape d) d'une itération précédente.

Le premier facteur de calibration peut également être choisi de façon à correspondre à la propriété auxiliaire mesurée sur l'échantillon.

Selon un mode de réalisation, la propriété optique auxiliaire de l'échantillon peut être mesurée en une pluralité d'éléments de surface (5i) de l'échantillon. Le procédé peut alors comporter une interpolation de ladite propriété optique auxiliaire à partir d'au moins deux propriétés optiques auxiliaires préalablement mesurées, de façon à établir une distribution spatiale, notamment bidimensionnelle, de ladite propriété optique auxiliaire.

Selon un mode de réalisation, la propriété optique auxiliaire de l'échantillon est mesurée selon au moins un élément de surface, ledit élément de surface étant déterminé à partir de la première image acquise lors de l'étape b).

Un autre objet de l'invention est un dispositif pour estimer des propriétés optiques d'un échantillon comme défini dans la revendication 8.

Le photodétecteur auxiliaire peut notamment être un capteur d'image. Il peut s'agir du premier capteur d'image.

Selon un mode de réalisation, la source de lumière auxiliaire est apte à former simultanément une pluralité de zones élémentaires d'illumination, espacées les unes des autres, à la surface de l'échantillon. Le dispositif peut alors comporter un masque, interposé entre la source de lumière auxiliaire et l'échantillon, le masque comportant des portions transparentes, permettant le passage du faisceau lumineux auxiliaire, et des portions opaques, bloquant le faisceau lumineux auxiliaire, de telle sorte que chaque zone élémentaire d'illumination est formée par une projection d'une portion transparente sur la surface de l'échantillon.

Selon un mode de réalisation, la source de lumière auxiliaire est apte à former successivement une pluralité de zones élémentaires d'illumination, espacées les unes des autres, à la surface de l'échantillon.

Selon un mode de réalisation, le microprocesseur est apte à mettre en œuvre les étapes c) et d) du procédé tel que décrit ci-dessus.

Un autre procédé d'estimation de propriétés optiques d'un échantillon comporte les étapes suivantes :

a) illumination de la surface de l'échantillon à l'aide d'un premier faisceau lumineux, produit par une première source de lumière ;
b) acquisition d'une première image d'un rayonnement lumineux rétrodiffusé par l'échantillon, ainsi illuminé, à l'aide d'un premier capteur d'image;
c) sélection d'une région d'intérêt dans la première image ;
d) détermination d'une pluralité de premiers facteurs de calibration, chaque premier facteur de calibration prenant la forme d'un ratio entre :

une modélisation d'une quantité de rayonnement rétrodiffusé par l'échantillon de calibration dont la première propriété optique est connue,
et une intensité représentative d'une image de calibration acquise, par ledit premier capteur d'image, lorsqu'un échantillon de calibration est illuminé par la première source de lumière,

de telle sorte qu'on dispose d'une pluralité de facteurs de calibration correspondant respectivement à des échantillons de calibration ayant des premières propriétés optiques de différentes valeurs, chaque premier facteur de calibration étant associé à une valeur de ladite première propriété optique ;
e) détermination d'une première grandeur d'intérêt, représentative d'une quantité de rayonnement rétrodiffusé par l'échantillon, dans ladite région d'intérêt, en multipliant une intensité représentative de la région d'intérêt par un premier facteur de calibration ;
f) estimation d'une première propriété optique en différentes régions d'intérêt de la surface de l'échantillon, à l'aide de ladite première grandeur d'intérêt ainsi déterminée;
g) réitération des étapes e) et f) en considérant, lors de l'étape e), un premier facteur de calibration correspondant à valeur de la première propriété optique estimée lors de l'étape f) de l'itération précédente, la réitération étant effectuée jusqu'à l'atteinte d'un critère de convergence.

Les étapes c) à g) peuvent être effectuées pour différentes régions d'intérêt de la première image, chaque région d'intérêt de l'image étant optiquement couplée à une région d'intérêt à la surface de l'échantillon.

Selon un mode de réalisation, le procédé comprend une mesure d'une propriété optique auxiliaire, telle que précédemment définie, sur un ou plusieurs éléments de surface de l'échantillon. Lors de l'étape e) la première grandeur d'intérêt peut être déterminée en considérant un premier facteur de calibration correspondant à ladite propriété optique auxiliaire.

Par premier facteur de calibration correspondant à une propriété optique, on entend un premier facteur de calibration représentatif d'un échantillon de calibration présentant ladite propriété optique. Un tel premier facteur de calibration peut être mesuré ou interpolé en se basant sur des facteurs de calibration mesurés.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1A représente une première composante d'un dispositif selon l'invention, pour estimer une propriété optique d'un échantillon à partir d'une première image, selon une première modalité.

La figure 1B représente une deuxième composante d'un dispositif selon l'invention, pour estimer une propriété optique auxiliaire d'un échantillon, selon une deuxième modalité.

La figure 2 illustre les étapes d'un procédé selon l'invention.

La figure 3A montre une image d'échantillons tests obtenue à l'aide de la configuration représentée sur la figure 1A.

La figure 3B représente une comparaison entre une première grandeur d'intérêt, mesurée à l'aide de la configuration représentée sur la figure 1A, et des modélisations de ladite première grandeur d'intérêt, chaque modélisation étant effectuée en fonction d'une valeur d'un coefficient d'absorption et d'un coefficient de diffusion réduit de l'échantillon.

La figure 3C montre des coefficients d'absorption d'un échantillon déterminés en mettant en œuvre les étapes décrites en lien avec la figure 2 dans trois longueurs d'onde différentes.

La figure 4A montre des estimations, à différentes longueurs d'onde, du coefficient d'absorption de quatre échantillons test en se basant sur une prise en compte arbitraire d'un coefficient de diffusion réduit. La figure 4B illustre les erreurs de mesure associées aux estimations présentées sur la figure 4A.

La figure 4C montre des estimations, à différentes longueurs d'onde, du coefficient d'absorption de quatre échantillons test en se basant sur une mesure d'un coefficient de diffusion réduit effectuée sur chaque échantillon. La figure 4D illustre les erreurs de mesure associées aux estimations présentées sur la figure 4C.

La figure 5A représente un mode de réalisation de la deuxième composante d'un dispositif selon l'invention.

Les figures 5B et 5C schématisent respectivement deux modes de réalisation différents d'un dispositif selon l'invention. La figure 5D montre un masque pouvant être appliqué dans le mode de réalisation exposé sur la figure 5C.

La figure 6 montre trois images pouvant être obtenues en mettant en œuvre l'invention.

## EXPOSE DE MODES DE RÉALISATION PARTICULIERS

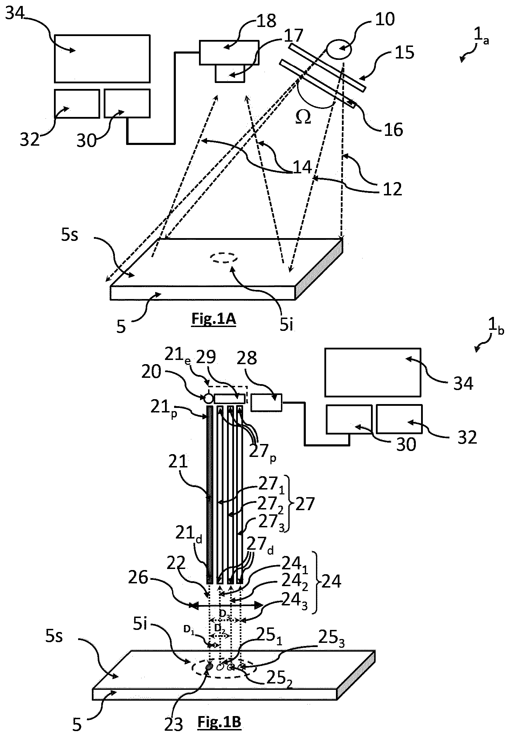

La figure 1A représente une première composante 1a d'un dispositif 1 permettant de former une image d'un échantillon et d'en déduire une propriété optique, ou une distribution spatiale d'une telle propriété optique.

Le terme propriété optique désigne une propriété gouvernant l'absorption ou la diffusion de la lumière dans l'échantillon. Il peut s'agir d'une propriété optique d'absorption, par exemple le coefficient d'absorption $\mu_a$, ou une propriété optique de diffusion par exemple un coefficient de diffusion réduit $\mu_s'$ ou un coefficient de diffusion $\mu_s$ ou un coefficient d'anisotropie de diffusion. Ces coefficients sont connus de l'homme du métier.

La première composante 1a permet d'appliquer une première modalité, dite d'imagerie spectrale de diffusion, à l'échantillon. D'une façon générale, selon cette première modalité, l'échantillon 5 est illuminé par une première source de lumière 10 et on forme une première image $I_1(\lambda)$ de ce dernier à l'aide d'un premier capteur d'image 18. La source de lumière peut être une source de lumière blanche, auquel cas le premier capteur d'image 18 est spectralement résolu, permettant l'acquisition d'images de l'échantillon selon différentes bandes spectrales $\lambda$. Par bande spectrale, on entend une longueur d'onde ou une plage de longueurs d'onde.

De façon alternative, la première source de lumière 10 peut émettre un premier faisceau lumineux 12, incident à l'échantillon, successivement selon différentes bandes spectrales $\lambda$, auquel cas le premier capteur d'image 18 acquiert autant d'images de l'échantillon que de bandes spectrales.

Selon l'exemple représenté, la première source de lumière 10 est une source de lumière blanche, associée à une roue à filtres 15, cette dernière comportant une pluralité de filtres dont la bande passante définit la bande spectrale $\lambda$ du premier faisceau lumineux 12, émis par la source et se propageant jusqu'à l'échantillon 5. La première source de lumière 10 peut être couplée à un diffuseur 16, permettant d'augmenter un angle solide $\Omega$ d'illumination de l'échantillon 5. De préférence, une part significative de la surface 5s de l'échantillon est illuminée par le premier faisceau lumineux 12, l'aire de la surface illuminée étant de préférence supérieure à 4 cm$^2$, voire supérieure à 10 cm$^2$. Cette surface est typiquement de l'ordre de 100 cm$^2$.

Sous l'effet de l'illumination par le premier faisceau lumineux 12, l'échantillon rétrodiffuse un rayonnement 14, dit premier rayonnement rétrodiffusé, ce dernier étant détecté par le premier capteur d'image 18, à travers un objectif 17.

Le dispositif comporte également un processeur 30, par exemple un microprocesseur, relié à une mémoire 32 et apte à exécuter des instructions stockées dans cette dernière, de façon à mettre en œuvre certaines étapes du procédé décrit ci-après. Le processeur peut être relié à un écran 34. Le processeur 30 effectue lesdites étapes à partir des premières images acquises par le capteur d'image 18 et/ou à partir de signaux de rétrodiffusion détectés par un photodétecteur auxiliaire 28 décrit en lien avec la figure 1B.

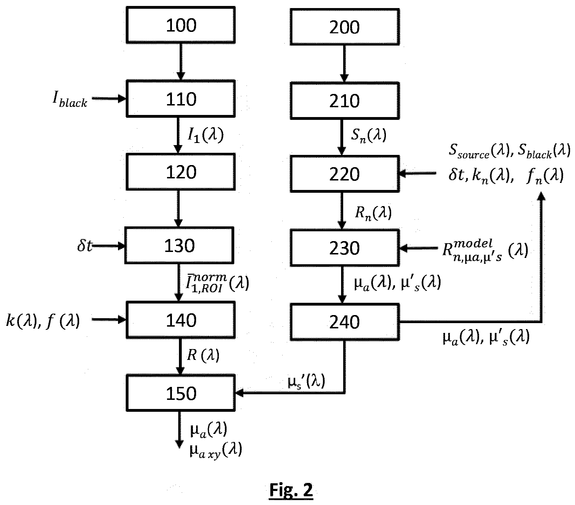

À partir de chaque première image $I_1(\lambda)$ acquise par le capteur d'image 18, dans une bande spectrale $\lambda$, on peut estimer des propriétés optiques de l'échantillon 5, dans ladite bande spectrale, comme décrit ci-après, en lien avec la figure 2.

La figure 1B représente une deuxième composante 1b du dispositif 1 permettant la mise en œuvre de l'invention selon une deuxième modalité dite de spectrométrie de réflectance diffuse (DRS). Comme décrit dans les documents relatifs à la DRS cités dans l'art antérieur, une source de lumière auxiliaire 20, par exemple une source de lumière blanche, est couplée à une fibre optique 21, dite fibre optique d'illumination, de façon à former un faisceau lumineux auxiliaire 22 se propageant vers l'échantillon 5. La fibre optique d'illumination s'étend entre une extrémité proximale $21_p$ disposée face à la source de lumière auxiliaire 20, collectant la lumière émise par cette dernière, et une extrémité distale $21_d$ disposée face à l'échantillon 5. L'extrémité distale $21_d$ peut être appliquée contre l'échantillon 5, ou être maintenue à distance de l'échantillon 5, comme représenté sur la figure 1B. Dans ce dernier cas, un système optique 26, par exemple un objectif ou une lentille, permet une focalisation du faisceau lumineux auxiliaire 22 sur la surface 5s de l'échantillon. Le système optique 26 peut présenter un facteur de grandissement inférieur ou supérieur à 1. Dans l'exemple représenté, le facteur de grandissement est égal à 1.

Le faisceau lumineux auxiliaire 22 forme, à la surface 5s de l'échantillon, une zone élémentaire d'illumination 23, correspondant à l'intersection du faisceau lumineux auxiliaire 22 avec ladite surface. Le terme zone élémentaire désigne une zone de forme délimitée et comportant une dimension inférieure ou égale à 1 cm, et de préférence inférieure ou égale à 1 mm. Par dimension, on entend un diamètre, une diagonale ou une largeur. L'aire d'une telle zone élémentaire est de préférence inférieure à 1 cm$^2$, voire inférieure à 1 mm$^2$. Sur l'exemple représenté sur la figure 1B, la zone élémentaire d'illumination 23 est située dans un plan focal image du système optique 26, tandis que l'extrémité distale $21_d$ de la fibre optique d'illumination 21 est située dans le plan focal objet de ce système optique. Le diamètre de la fibre optique d'illumination 21 est généralement compris entre 50 μm et 1 mm, et est par exemple égal à 500 μm.

Le dispositif comporte une pluralité de fibres optiques de détection 27, chacune s'étendant entre une extrémité distale $27_d$, faisant face à l'échantillon, et une extrémité proximale $27_p$. Chaque fibre de détection 27 est apte à collecter un rayonnement rétrodiffusé 24, dit deuxième rayonnement rétrodiffusé, par l'échantillon 5 lorsque ce dernier est illuminé par le faisceau lumineux auxiliaire 22. Les extrémités distales $27_d$ de chaque fibre de détection 27 sont disposées à des distances différentes de l'extrémité distale $21_d$ de la fibre d'illumination 21, de façon à ce que chaque fibre de détection soit apte à collecter un rayonnement rétrodiffusé par l'échantillon, émanant de la surface de l'échantillon à une distance $D_n$ de la zone d'illumination 23, dite distance de rétrodiffusion, différente l'une de l'autre.

Le principe de cette modalité est de déterminer une quantité de photons rétrodiffusés par l'échantillon selon une ou, de préférence plusieurs distances de rétrodiffusion $D_n$. Pour cela, chaque fibre optique de détection 27 collecte un rayonnement rétrodiffusé 24 émanant d'une zone élémentaire de détection 25, cette dernière s'étendant à une distance de rétrodiffusion $D_n$ de la zone élémentaire d'illumination 23. Le photodétecteur auxiliaire 28 détecte un signal rétrodiffusé $S_n$ dont l'intensité dépend d'une quantité de photons rétrodiffusés à la distance de rétrodiffusion $D_n$.

Sur la figure 1B, on a représenté trois fibres de détection 27 :

- une première fibre de détection $27_1$, apte à collecter un rayonnement de diffusion $24_1$ émanant de la surface de l'échantillon au niveau d'une zone élémentaire de détection $25_1$ s'étendant à une première distance de rétrodiffusion $D_1$ par rapport à la zone élémentaire d'illumination 23.
- Une deuxième fibre de détection $27_2$, apte à collecter un rayonnement de diffusion $24_2$ émanant de la surface de l'échantillon au niveau d'une zone élémentaire de détection $25_2$ s'étendant à une deuxième distance de rétrodiffusion $D_2$ par rapport à la zone élémentaire d'illumination 23.
- Une troisième fibre de détection $27_3$, apte à collecter un rayonnement de diffusion $24_3$ émanant de la surface de l'échantillon au niveau d'une zone élémentaire de détection $25_3$ s'étendant à une troisième distance de rétrodiffusion $D_3$ par rapport à la zone élémentaire d'illumination 23.

Les première, deuxième et troisième distances de rétrodiffusion sont non nulles et différentes les unes des autres. Selon le facteur de grandissement du système optique 26, elles peuvent s'étendre de quelques dizaines de $\mu$m à quelques mm, voire cm de la zone élémentaire d'illumination 23. Les zones élémentaires de rétrodiffusion 25 sont de préférence séparées de la zone élémentaire d'illumination 23, le terme séparé signifiant qu'il n'y a pas d'intersection entre une zone élémentaire de rétrodiffusion 25 et la zone élémentaire d'illumination 23.

Plusieurs fibres optiques de détection peuvent être conjuguées à des zones élémentaires de détection s'étendant à une même distance de rétrodiffusion de la zone élémentaire d'illumination. Dans ce cas, ces fibres peuvent être disposées selon un cercle autour de la zone élémentaire d'illumination. Cela permet d'augmenter la quantité de rayonnement de rétrodiffusion détecté, correspondant à une même distance de rétrodiffusion. On améliore ainsi la sensibilité de la mesure. Cela permet également d'obtenir d'effectuer une moyenne des rayonnements rétrodiffusés à chaque distance de rétrodiffusion. De telles configurations, dites concentriques, sont décrites dans le document EP2762064 ou dans la publication « Sorgato 2015 » précédemment citée.

L'extrémité proximale $27_p$ de chaque fibre optique de détection est couplée à un commutateur optique 29, permettant un couplage sélectif de chaque fibre optique avec un photodétecteur auxiliaire 28, ce dernier étant, dans cet exemple, un spectrophotomètre. Le spectrophotomètre est apte à former un spectre en longueur d'onde de chaque rayonnement rétrodiffusé selon une distance de rétrodiffusion. Le processeur 30 est apte à traiter les spectres de façon à estimer une propriété optique auxiliaire de l'échantillon, comme expliqué en lien avec la figure 2.

Une différence notable entre la première composante 1a du dispositif, représentée sur la figure 1A et la deuxième composante 1b du dispositif, représentée sur la figure 1B, concerne les extensions spatiales du premier faisceau lumineux 12 et du faisceau lumineux auxiliaire 22. Comme précédemment évoqué, la surface de l'échantillon éclairée par le premier faisceau lumineux 12 est supérieure à quelques $cm^2$, et le plus souvent supérieur 5 $cm^2$, voire 10 ou 100 $cm^2$, tandis que la surface de l'échantillon éclairée par le faisceau lumineux auxiliaire 22 est inférieure à 1 $cm^2$. La première modalité permet d'effectuer une détermination d'une propriété optique simultanément sur une surface importante de l'échantillon, c'est-à-dire sur différentes régions d'intérêt de la surface 5s de l'échantillon, tandis que la deuxième modalité permet une mesure quantitative d'une propriété optique locale de l'échantillon. Ainsi, la première modalité permet d'obtenir, à partir d'une même image $I_1(\lambda)$, une distribution spatiale bidimensionnelle d'une propriété optique, ce que ne permet pas la deuxième modalité, sauf à multiplier le nombre de fibres optiques d'illumination et de fibres optiques de détection, comme décrit dans la publication « Nichols 2015» précédemment citée.

On va à présent décrire les principales étapes d'un procédé de détermination de propriétés optiques d'un échantillon, en lien avec la figure 2. Les étapes 100 à 150 concernent la première modalité, en l'occurrence l'imagerie spectrale de diffusion, tandis que les étapes 200 à 240 sont concernent la deuxième modalité, en l'occurrence la spectrométrie de réflectance diffuse.

Étape 100 : illumination de l'échantillon avec la première source de lumière.

Au cours de cette étape, l'échantillon est illuminé avec la source de lumière blanche 10, émettant un premier faisceau lumineux 12 se propageant jusqu'à l'échantillon dans une bande spectrale $\lambda$ ajustée par la roue à filtres 15. Dans cet exemple, la source de lumière utilisée est une source halogène Schott de référence KL 2500 LCD. Elle est associée à une roue à filtre 15 comportant trois filtres définissant des bandes spectrales $\lambda$ respectivement centrées sur les longueurs d'onde 500 nm, 600 nm et 700 nm. La largeur de bande de chaque filtre, correspondant à la largeur à mi-hauteur du pic d'émission, est d'environ 10 nm. Le diffuseur 16 est un anneau au niveau duquel aboutissent des guides de lumière provenant de la première source de lumière 10. Il permet de répartir la lumière, de façon la plus uniforme possible, selon un angle solide d'émission $\Omega$.

Les étapes 110 et 150 ci-dessous sont décrites en référence à une bande spectrale, sachant qu'elles peuvent être mises en œuvre successivement selon chaque bande spectrale du premier faisceau 12 émis par la première source de lumière et atteignant l'échantillon.

Étape 110 : Acquisition d'une image.

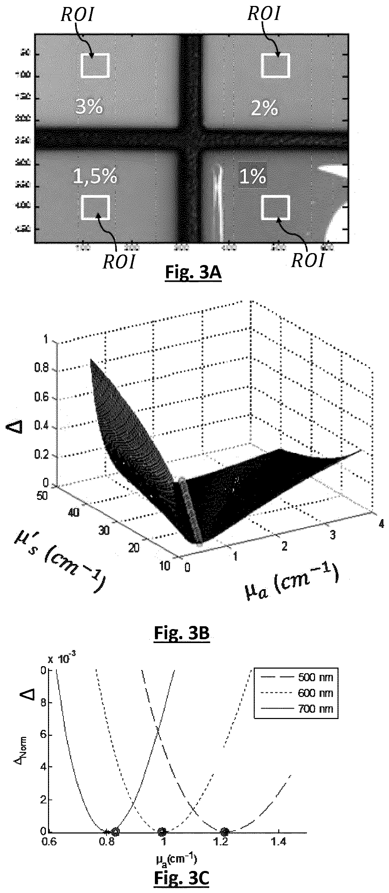

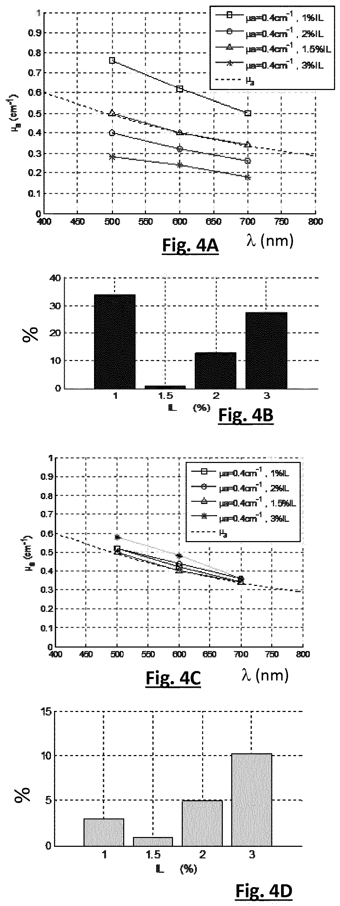

Le premier capteur d'image acquiert une première image $I_1(\lambda)$ d'un rayonnement 14 formé par une rétrodiffusion, par l'échantillon 5 du premier faisceau lumineux 12, dans une longueur d'onde ou une bande spectrale $\lambda$. La figure 3A représente une telle image, l'échantillon étant formé de différents échantillons test, présentant des propriétés de diffusion optique différentes, et décrits en lien avec les figures 4A à 4D.

L'image acquise $I_1(\lambda)$ est de préférence corrigée d'une image de noir $I_{black}$, obtenue dans le noir, selon une même durée d'acquisition, la première source de lumière étant éteinte. L'image de noir $I_{black}$ est représentative du bruit du premier capteur d'image et d'un niveau d'obscurité du dispositif. Elle est également de préférence normalisée par une image dite de blanc $I_{flatfield}$, également désignée par le terme anglosaxon « flat field », destinée à corriger les hétérogénéités spatiales de l'illumination de l'échantillon et de l'efficacité de détection de l'image. En effet, l'éclairement de l'échantillon par la première source de lumière peut ne pas être homogène. De plus, en particulier du fait de composants optiques dans l'objectif 17, l'efficacité de détection n'est pas homogène, notamment en raison d'effets de vignettage à la périphérie de l'image. L'image acquise est normalisée par l'image de blanc $I_{flatfield}$, cette dernière étant obtenue en effectuant une image d'un échantillon dont la rétrodiffusion est considérée comme homogène, et en normalisant l'image ainsi obtenue par la valeur d'intensité du pixel le plus intense de ladite image. Le recours à une telle image de blanc est une pratique courante.

Étape 120 : Segmentation de la première image en régions d'intérêt et sélection d'une région d'intérêt.

Au cours de cette étape, on sélectionne une ou plusieurs régions d'intérêt ROI dans la première image $I_1(\lambda)$ obtenue au cours de l'étape 110. Une région d'intérêt peut correspondre à différents pixels adjacents de l'image présentant un même niveau d'intensité, ou un niveau d'intensité comparable. Chaque région d'intérêt de l'image est associée à une région d'intérêt de l'échantillon, à laquelle elle est couplée par l'objectif 17. Dans l'image de la figure 3A, on a représenté 4 régions d'intérêt, chacune étant délimitée par un rectangle. Chaque région d'intérêt correspond à un échantillon test décrit en lien avec les figures 4A à 4D. Une telle sélection permet d'éviter des artéfacts dus notamment à la réflexion spéculaire. Une région d'intérêt peut ne comprendre qu'un seul pixel. Elle peut également regrouper des pixels non adjacents mais présentant une même intensité ou une intensité comparable.

Une région d'intérêt peut également correspondre à une région de l'échantillon dans laquelle on considère, de façon arbitraire ou sur la base de mesures préalables, qu'une propriété optique de l'échantillon est homogène, ou qu'une composition de l'échantillon est homogène.

Étape 130 : Détermination d'une intensité moyenne dans chaque région d'intérêt.

Au cours de cette étape, on détermine une grandeur d'intérêt représentative de chaque région d'intérêt. Si $I_{1,ROI}(\lambda)$ désigne l'image de la région d'intérêt ROI, on effectue une moyenne de l'intensité des pixels dans cette région d'intérêt, de façon à obtenir une intensité moyenne $\overline{I}_{1,ROI}(\lambda)$.

Cette intensité moyenne peut être normalisée par une durée d'acquisition $\delta t$ de l'image, de telle sorte que

$$\overline{I}_{1,ROI}^{norm}(\lambda) = \frac{\overline{I}_{1,ROI}(\lambda)}{\delta t}$$, (1) l'exposant *norm* désignant le fait que l'image est normalisée par la durée d'acquisition.

Etape 140 : Prise en compte de facteurs correctifs.

Cette étape consiste à appliquer un facteur de calibration $f(\lambda)$, dit premier facteur de calibration, à l'intensité moyenne. Ce premier facteur de calibration prend en compte l'intensité du premier faisceau lumineux 12 incident à l'échantillon, ainsi que la sensibilité du premier capteur d'image 18. Il est déterminé au cours d'une phase de calibration, mettant en œuvre un échantillon de calibration, ou fantôme de calibration, dont les propriétés optiques sont connues.

Le premier facteur de calibration $f(\lambda)$ correspond à un ratio entre une réflectance modélisée $R_{calib}^{model}(\lambda)$ de l'échantillon de calibration et l'intensité moyenne d'une image $\overline{I}_{calib}^{norm}(\lambda)$ de l'échantillon acquise par le dispositif, en conservant les mêmes paramètres expérimentaux que lors de l'analyse de l'échantillon : mêmes réglages de la source de lumière 10 et du capteur d'image 18, même distance de l'échantillon de calibration vis-à-vis de la source et du capteur d'image, même niveau obscurité dans lequel est placé l'échantillon.

$$f(\lambda) = \frac{R_{calib}^{model}(\lambda)}{\overline{I}_{calib}^{norm}(\lambda)} (2)$$

Le premier facteur de calibration est tel que

Par ailleurs, certains paramètres du dispositif peuvent évoluer entre l'instant $t_0$ ou la calibration précédemment décrite a été réalisée et l'instant t de la mesure sur l'échantillon examiné. Il peut par exemple s'agir de la variation de l'intensité de la source de lumière, de la réponse des guides de lumière, ou une variation de la sensibilité du capteur d'image, et, d'une façon plus générale, de différents composants du dispositif. Afin de prendre en compte cette variabilité,

$$k(\lambda) = \frac{I_{t-ref}(\lambda)}{I_{t0-ref}(\lambda)}$$

un premier facteur de correction $k(\lambda)$ est appliqué, de telle sorte que , (3) où $I_{t-ref}(\lambda)$ et $I_{t0-ref}(\lambda)$ désignent respectivement une intensité, mesurée par le capteur d'image 18, d'un fantôme de référence respectivement à l'instant de la mesure t et à un instant initial $t_0$. Le fantôme de référence peut être un échantillon de calibration ou un fantôme comportant une surface dont la réflectance est constante. Il peut par exemple s'agir d'une pastille appliquée sur un support que l'on dispose face au capteur d'image 18 avant d'effectuer les mesures. Les mesures du fantôme de référence, à l'instant initial $t_0$ et à l'instant de la mesure t sont effectuées selon les mêmes paramètres expérimentaux.

L'application du premier facteur de calibration $f(\lambda)$ et du premier facteur de correction $k(\lambda)$ permet d'obtenir une réflectance $R(\lambda)$ de l'échantillon analysé 5, de telle sorte que :

$$R(\lambda) = \overline{I}_{1,ROI}^{norm}(\lambda) \times k(\lambda) \times f(\lambda) (4)$$

Ainsi, on obtient, à partir de la première image, une première grandeur d'intérêt $R(\lambda)$ représentative de la réflectance de l'échantillon, dans la région d'intérêt considérée, cette réflectance représentant une quantité de rayonnement rétrodiffusé par ladite région d'intérêt normalisée par une intensité du premier faisceau lumineux 12.

Étape 150 : estimation d'une première propriété optique

Au cours de l'étape 150, on cherche à estimer une première propriété optique $p_1$ de l'échantillon, par exemple le coefficient d'absorption $\mu_a(\lambda)$, dans la bande spectrale $\lambda$ du premier faisceau lumineux 12.

Pour cela, on utilise une modélisation, dans la bande spectrale considérée, de la réflectance de l'échantillon, cette modélisation prenant en compte une pluralité de propriétés optiques $p$, parmi lesquelles le coefficient d'absorption $\mu_a$, le coefficient de diffusion réduit $\mu'_s$. On effectue des modélisations en faisant varier le coefficient d'absorption et le coefficient de diffusion réduit respectivement entre une valeur minimale et une valeur maximale. On obtient ainsi une réflectance modélisées $R^{model}_{(\mu_a,\mu'_s)}(\lambda)$ pour chaque valeur de $\mu_a$ et de $\mu'_s$, considérées. La modélisation peut être réalisée sur la base d'un modèle analytique ou en utilisant une méthode stochastique de type Monte Carlo.

On peut effectuer une comparaison, par exemple sous la forme d'un écart quadratique entre la réflectance mesurée $R(\lambda)$ et chaque réflectance modélisée $R^{model}_{(\mu_a,\mu'_s)}(\lambda)$ selon l'expression :

$$\Delta(\mu_a(\lambda), \mu'_s(\lambda)) = \sqrt{\left(R(\lambda) - R^{model}_{(\mu_a,\mu'_s)}(\lambda)\right)^2} \quad (5)$$

$$\text{avec } (\mu_a(\lambda), \mu'_s(\lambda)) = \operatorname*{argmin}_{\mu_a(\lambda),\mu'_s(\lambda)} \left( \sqrt{\left(R(\lambda) - R^{model}_{(\mu_a,\mu'_s)}(\lambda)\right)^2} \right) \quad (5')$$

On a représenté, sur la figure 3B, une telle comparaison $\Delta(\mu_a(\lambda), \mu'_s(\lambda))$ en fonction de $(\mu_a(\lambda)$ et $\mu'_s(\lambda)$ en se basant sur une image mesurée sur un échantillon test dans une bande spectrale centrée sur 600 nm. Les propriétés optiques $(\mu_a(\lambda), \mu'_s(\lambda))$ de l'échantillon sont celles minimisant la différence $\Delta(\mu_a(\lambda), \mu'_s(\lambda))$. Cependant, cette différence ne prend pas une valeur minimum pour un point de l'espace $(\mu_a(\lambda), \mu'_s(\lambda))$, mais selon une multitude de points, formant une ligne. Ainsi, le problème est indéterminé, puisqu'il ne comporte pas une solution, c'est-à-dire un couple $(\mu_a(\lambda), \mu'_s(\lambda))$ mais une pluralité, voire une infinité de solutions. Afin d'estimer une de ces deux propriétés optiques, par exemple $\mu_a(\lambda)$, la valeur de l'autre propriété optique, en l'occurrence $\mu'_s(\lambda)$, doit être préalablement établie.

Selon l'art antérieur, pour corriger cette indétermination, on considère une valeur prédéterminée du coefficient de diffusion réduit $\mu'_s$. Dans la publication Bjorgan 2014, le coefficient $\mu'_s$ est fixé selon un modèle de diffusion analytique, basé sur des échantillons considérés comme représentatifs de l'échantillon analysé.

Les inventeurs ont constaté qu'une telle démarche conduit à une erreur de mesure pouvant être rédhibitoire, comme décrit ci-après en lien avec les figures 4A et 4B. Ils en ont conclu que pour lever cette indétermination, il est préférable non pas d'utiliser une valeur prédéterminée du coefficient de diffusion réduit, censée être représentative de l'échantillon examiné, mais de mesurer cette valeur directement sur l'échantillon examiné.

Pour cela, une deuxième modalité est utilisée, visant à estimer, au moins localement, une propriété optique auxiliaire de l'échantillon 5, en l'occurrence le coefficient de diffusion réduit $\mu'_s$.

Autrement dit, la méthode proposée vise à estimer une première propriété optique de l'échantillon, par exemple $\mu_a$, dans une bande spectrale, à l'aide d'une première modalité, cette estimation étant basée sur une mesure d'une propriété optique auxiliaire de l'échantillon, par exemple $\mu'_s$, dans ladite bande spectrale, par le biais d'une deuxième modalité de mesure indépendante de la première modalité.

Dans cet exemple, la propriété optique auxiliaire est mesurée par une méthode de type DRS, visant à former une zone élémentaire d'illumination 23 sur la surface 5s de l'échantillon 5, et à détecter au moins un rayonnement diffusé et de préférence au moins deux rayonnements rétrodiffusés $24_1$, $24_2$, $24_3$ respectivement selon deux distances de rétrodiffusion $D_1$, $D_2$, $D_3$ non nulles et différentes l'une de l'autre. Cette méthode est appliquée localement, sur au moins un élément de la surface 5i de la surface 5s, comme représenté sur la figure 1B. Le dispositif mis en œuvre peut être celui décrit en lien avec la figure 1B, et appliqué selon la méthode décrite dans la publication « Sorgato 2015 » dont les principales étapes sont les suivantes :

Étape 200 : Illumination

Au cours de cette étape, on illumine l'échantillon en activant la source de lumière auxiliaire 20, de façon à diriger un faisceau lumineux auxiliaire, ou deuxième faisceau lumineux 22 vers la surface 5s de l'échantillon 5, l'intersection du faisceau lumineux auxiliaire 22 avec la surface 5s de l'échantillon formant une zone élémentaire d'illumination 23. La source de lumière peut être par exemple une lampe tungstène halogène de référence HL 2000 de Océan Optics.

Étape 210 : Détection de signaux rétrodiffusés selon différentes distances de rétrodiffusion.

Au cours de cette étape, le photodétecteur auxiliaire 28 détecte au moins deux signaux rétrodiffusés $24_1$, $24_2$ selon deux distances de rétrodiffusion $D_1$, $D_2$ différentes. Dans cet exemple, le photodétecteur auxiliaire 28 est un spectromètre de type MAYA2000 Pro de Ocean Optics. On forme alors, à chaque distance de rétrodiffusion $D_n$, un

signal détecté, dit signal de rétrodiffusion, $S_n(\lambda)$ dans la bande spectrale $\lambda$, l'indice $n$ désignant la distance de rétrodiffusion. Chaque signal de rétrodiffusion $S_n(\lambda)$ est de préférence corrigé d'un signal de noir $S_{black}$, mesuré dans le noir, selon une même durée d'acquisition, la source de lumière auxiliaire étant éteinte. Le signal de noir $S_{black}$ est représentatif du bruit du photodétecteur auxiliaire 28 et d'un niveau d'obscurité dans lequel l'échantillon est placé.

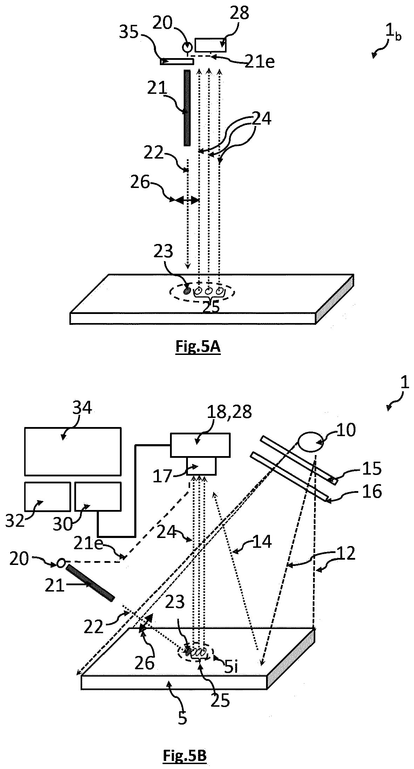

De façon alternative, l'échantillon peut être successivement illuminé, par la source de lumière auxiliaire 20, selon différentes bandes spectrales $\lambda$, le photodétecteur auxiliaire 28 n'étant pas résolu spectralement. Il peut en particulier s'agir d'un capteur d'image, comme décrit en lien avec les figures 5A à 5C.

Étape 220 : Normalisation et application de facteurs correctifs pour obtenir une grandeur d'intérêt auxiliaire, ou deuxième grandeur d'intérêt.

Au cours de cette étape, chaque signal de rétrodiffusion détecté $S_n(\lambda)$, corrigé par le signal de noir $S_{black}$, est normalisé par une durée d'acquisition $\delta t$ et par une quantité de lumière $S_{source}(\lambda)$ émise par la source de lumière auxiliaire 20. Une telle quantité de lumière peut être mesurée en disposant une fibre optique, dite de retour d'excitation 21e, entre la source de lumière auxiliaire 20 et le photodétecteur auxiliaire 28.

On applique également un facteur de calibration, dit deuxième facteur de calibration, $f_n(\lambda)$ à chaque signal de rétrodiffusion $S_n(\lambda)$ ainsi corrigé et normalisé. Ce deuxième facteur de calibration, dépendant de la distance de rétrodiffusion $D_n$ prend en compte l'effet de différents composants de la fibre d'illumination 21 et de la fibre de détection $27_n$ sur le signal de rétrodiffusion. De façon analogue au premier facteur de calibration $f(\lambda)$ décrit dans l'étape 140, chaque deuxième facteur de calibration est déterminé au cours d'une phase de calibration, mettant en œuvre un échantillon de calibration dont les propriétés optiques sont connues. Ce facteur de calibration correspond à un ratio entre une réflectance modélisée $R_{calib,n}^{model}(\lambda)$ de l'échantillon de calibration à la distance de rétrodiffusion $D_n$ et une réflectance $R_{calib,n}(\lambda)$ de l'échantillon de calibration mesurée par le dispositif, à la même longueur d'onde et à la même distance de rétrodiffusion, en conservant les mêmes paramètres expérimentaux qu'avec l'échantillon analysé : mêmes réglages de la source 20 et du photodétecteur 28, même distance de l'échantillon de calibration vis-à-vis de la source et du photodétecteur.

Le deuxième facteur de calibration est tel que
$$f_n(\lambda) = \frac{R_{calib,n}^{model}(\lambda)}{R_{calib,n}(\lambda)} \quad (6)$$
avec :

$$R_{calib,n}(\lambda) = \frac{S_{calib,n}(\lambda) - S_{black}(\lambda)}{S_{source}(\lambda) \times \delta t_{calib}} \quad (6'),$$

$S_{calib,n}(\lambda)$ désignant le signal de rétrodiffusion, à une distance de rétrodiffusion $D_n$, mesuré par le photodétecteur auxiliaire 28 lorsque l'échantillon mesuré est l'échantillon de calibration, $\delta t_{calib}$ désignant la durée de l'acquisition du signal de rétrodiffusion $S_{calib,n}(\lambda)$.

Par ailleurs, certains paramètres du dispositif peuvent évoluer entre l'instant $t_0$ auquel la calibration précédemment décrite a été réalisée et l'instant t de la mesure. Il peut par exemple prendre en compte une variation de la sensibilité du photodétecteur auxiliaire 28 ou le vieillissement des fibres optiques de détection 27.

Afin de prendre en compte cette variabilité, un facteur de correction, dit deuxième facteur de correction, $k_n(\lambda)$ peut être appliqué, de telle sorte que
$$k_n(\lambda) = \frac{f_{t,n}(\lambda)}{f_{t0,n}(\lambda)} \quad (7)$$
où $f_{t,n}(\lambda)$ et $f_{t0,n}(\lambda)$ représentent respectivement des deuxièmes facteurs de calibration mesurés aux instants t et $t_0$.

Cette étape permet d'obtenir une réflectance de l'échantillon $R_n(\lambda)$, à la distance de rétrodiffusion $D_n$ et à la longueur d'onde $\lambda$, selon l'expression :

$$R_n(\lambda) = \frac{S_n(\lambda) - S_{black}(\lambda)}{S_{source}(\lambda) \times \delta t} \times f_n(\lambda) \times k_n(\lambda) \quad (8)$$

Cette réflectance correspond à une grandeur d'intérêt auxiliaire, représentative d'une intensité d'un rayonnement rétrodiffusé à une distance de rétrodiffusion $D_n$, non nulle, par rapport à la zone d'illumination élémentaire 23. Ce rayonnement rétrodiffusé peut également être désigné par le terme « deuxième rayonnement rétrodiffusé ».

Étape 230 : Détermination des propriétés optiques locales de l'échantillon

Pour au moins une longueur d'onde $\lambda$, et en considérant au moins autant de distances de rétrodiffusion $D_n$ différentes qu'il y a de propriétés optiques à estimer, on détermine les propriétés optiques $p$ présentant le moindre écart entre la réflectance $R_n(\lambda)$, déterminée lors de l'étape 220, à la longueur d'onde $\lambda$, et une réflectance modélisée

$R_{n,p}^{model}(\lambda)$, cette réflectance étant modélisée en considérant une pluralité de valeurs desdites propriétés optiques $p$, à ladite distance de rétrodiffusion $D_n$. Cette détermination peut être réalisée par la minimisation d'un écart quadratique, et par exemple selon l'expression :

$$p = \underset{p}{argmin}\left(\sum_{n=1}^{N}\left(R_{n,p}^{model}(\lambda) - R_n(\lambda)\right)^2\right) \quad (9)$$

- N désigne le nombre de distances de rétrodiffusion prises en compte,
- $R_{n,p}^{model}$ est une réflectance modélisée, à la distance de rétrodiffusion $D_n$, en prenant en compte des valeurs prédéterminées d'au moins une propriété optique $p$. Le paramètre p peut correspondre à une propriété optique, ou un ensemble de propriétés optiques.

Dans cet exemple, les propriétés optiques recherchées sont $\mu_a(\lambda)$ et $\mu'_s(\lambda)$. Ainsi, le couple $(\mu_a(\lambda), \mu'_s(\lambda))$ recherché est celui présentant le moindre écart entre la réflectance mesurée $R_n(\lambda)$, à la longueur d'onde $\lambda$, et une réflectance modélisée $R_{n,\mu a,\mu s'}^{model}(\lambda)$ pour différentes valeurs de $\mu_a(\lambda)$ et de $\mu'_s(\text{Å})$, à ladite distance de rétrodiffusion $D_n$. Cette détermination peut être réalisée selon l'expression :

$$\left(\mu_a(\lambda), \mu'_s(\lambda)\right) = argmin_{\left(\mu_a(\lambda),\mu'_s(\lambda)\right)}\left(\sum_{n=1}^{N}\left(R_{n,\mu a,\mu s'}^{model}(\lambda) - R_n(\lambda)\right)^2\right) \quad (9'),$$

$R_{n,\mu a,\mu s}^{model}(\lambda)$ désignant une réflectance modélisée, à la distance de rétrodiffusion $D_n$, en considérant différentes valeurs de $\mu_a(\lambda)$ et $\mu_s'(\lambda)$.

Les différentes valeurs de réflectance modélisée $R_{n,\mu a,\mu s'}^{model}(\lambda)$ sont obtenues, pour une pluralité de couples de valeurs $\mu_a(\lambda)$, $\mu'_s(\lambda)$ au cours d'une phase de paramétrage, par simulation numérique mettant en oeuvre une méthode de type Monte Carlo ou par un modèle analytique.

Etape 240 : transmission de la propriété optique auxiliaire ou réitération.

Au cours de cette étape, la propriété optique auxiliaire, en l'occurrence le coefficient de diffusion réduit $\mu'_s(\lambda)$, déterminée lors de l'étape 230, est transmise pour être utilisée pour le calcul de la première propriété optique. Les propriétés optiques déterminées lors de l'étape 230 peuvent également être utilisées pour sélectionner un deuxième facteur de calibration, utilisé lors d'une nouvelle itération de l'étape 220, comme décrit ci-après.

De préférence, lors de l'étape 220, on dispose d'une pluralité de deuxièmes facteurs de calibration $f_n(\lambda)$, réalisés en utilisant des échantillons de calibration dont les propriétés optiques $p$, en particulier $\mu_a(\lambda)$, et/ou $\mu'_s(\lambda)$, sont différentes. On peut également calculer des facteurs de calibration par interpolation entre deux facteurs de calibration respectivement obtenus avec deux échantillons de calibration présentant des propriétés optiques différentes. On dispose alors de deuxièmes facteurs de calibration, notés $f_n^p(\lambda)$, associés à chaque distance de rétrodiffusion $D_n$ et à différentes valeurs des propriétés optiques $p$. Lors d'une première itération de l'étape 220, on utilise un deuxième facteur de calibration $f_n^p(\lambda)$ correspondant à des propriétés optiques définies de façon arbitraire. Suite à l'étape 240, l'étape 220 peut être réitérée, en prenant en compte un deuxième facteur de calibration $f_n^p(\lambda)$ correspondant aux propriétés optiques $p$ déterminées lors de l'étape 230. Le procédé peut être réitéré plusieurs fois, mais les inventeurs estiment que deux itérations sont généralement suffisantes. Le deuxième facteur de correction $k_n$ peut n'être déterminé que sur un seul échantillon de calibration et utilisé quel que soit deuxième le facteur de calibration $f_n^p(\lambda)$ appliqué. L'adaptation du deuxième facteur de calibration à des propriétés optiques de l'échantillon déterminées au cours d'une itération précédente, méthode dite du facteur de calibration adaptatif, a été décrite dans la publication Sorgato 2015. Cela permet de limiter les erreurs de mesures dues à la prise en compte d'un facteur de calibration non représentatif des propriétés optiques réelles de l'échantillon examiné.

Ainsi, l'étape 230 permet de mesurer, localement, une propriété optique auxiliaire de diffusion, en l'occurrence

$\mu'_s(\lambda)$, cette propriété étant utilisée, dans l'étape 150, pour estimer la première propriété optique, en l'occurrence $\mu'_a(\lambda)$ à l'aide de la première modalité. Il est préférable que la bande spectrale $\lambda$, à laquelle la propriété optique auxiliaire est mesurée, soit proche ou incluse dans la bande spectrale $\lambda$ à laquelle la première propriété optique est estimée. La mesure de la propriété optique auxiliaire $\mu'_s(\lambda)$ permet de lever l'indétermination évoquée en lien avec l'étape 150, et d'estimer, au cours cette étape, la valeur $\mu_a(\lambda)$ minimisant la fonction d'écart $\Delta(\mu_a, \mu'_s)$ mentionnée en lien avec l'équation (5).

Les étapes 100 à 150 sont de préférence mises en œuvre pour différentes zones d'intérêt ROI de la première image, ce qui permet d'estimer la première propriété optique en chacune de ces zones d'intérêt. On obtient alors une estimation de la première propriété optique en différents points de la surface de l'échantillon. Une région d'intérêt peut correspondre à un pixel ou à un groupe de pixels de la première image. Un groupe de pixels peut être composé de pixels de même intensité, ou d'intensité située dans une même plage de valeurs. Un groupe de pixels peut également résulter d'un maillage arbitraire de la surface de l'échantillon en différentes régions d'intérêt ROI.

La figure 3C illustre des fonctions d'écart $\Delta(\mu_a(\lambda), \mu'_s(\lambda))$, telles qu'explicitées selon l'équation (5), obtenues en utilisant un premier échantillon de test IL1-1,5%, dans trois longueurs d'onde : $\lambda = 500$ nm, $\lambda = 600$ nm et $\lambda = 700$ nm, le coefficient $\mu'_s(\lambda)$ de l'échantillon de test ayant été mesuré, à ces trois longueurs d'onde. Le premier échantillon de test IL1-1.5% est un échantillon comportant une concentration d'encre de Chine permettant d'obtenir un coefficient d'absorption $\mu_a(\lambda)$ égal à 1 cm$^{-1}$ à $\lambda=600$ nm, et comportant une concentration volumique de 1.5% d'intralipide. Cette fonction d'écart ne comporte qu'une inconnue, à savoir $\mu_a(\lambda)$. Pour chaque longueur d'onde, la valeur $\mu_a(\lambda)$ minimisant la fonction d'écart est le coefficient d'absorption de l'échantillon. Sur la figure 3C, on a représenté les valeurs estimées, représentées par un cercle, ces dernières étant proches des valeurs réelles, représentées par un astérisque *.

Dans ce qui précède, on estime une propriété d'absorption $\mu_a(\lambda)$ (première propriété optique) à l'aide de la modalité d'imagerie spectrale de réflectance (MSI -1$^{re}$ modalité), tandis qu'on estime une propriété de diffusion $\mu'_s(\lambda)$ (propriété otique auxiliaire) à l'aide de spectrométrie de réflectance diffuse (DRS - 2$^e$ modalité) appliquée sur l'échantillon. De façon alternative, on pourrait estimer une propriété de diffusion $\mu'_s(\lambda)$ (première propriété optique) par imagerie spectrale de réflectance et mesurer une propriété d'absorption $\mu_a(\lambda)$ (propriété optique auxiliaire) par spectrométrie de réflectance diffuse. Cependant, la profondeur de champ de la DRS est plus élevée pour la mesure d'un coefficient de diffusion que pour la mesure d'un coefficient d'absorption, cette profondeur de champ étant d'environ 2mm pour la mesure de $n'_s(\lambda)$ et d'environ 500$\mu$m pour la mesure de $\mu_a(\lambda)$, avec le dispositif de mesure par DRS décrit ci-avant. Ainsi, la mesure d'un coefficient d'absorption par DRS suppose une mise au point particulièrement précise du dispositif représenté sur la figure 1B. Cette contrainte de mise au point est moindre lorsqu'on utilise la DRS pour déterminer un coefficient de diffusion $\mu'_s(\lambda)$. Par ailleurs, la profondeur de champ de l'imagerie spectrale de réflectance, appliquée à la détermination de $\mu_a(\lambda)$ est plus élevée que la profondeur de champ de la DRS, avec le dispositif décrit ci-avant. Pour ces raisons de profondeur de champ, il est préférable de recourir à la DRS pour mesurer une propriété optique de diffusion $\mu'_s(\lambda)$, et à la MSI pour mesurer une propriété optique d'absorption $\mu_a(\lambda)$.

Par ailleurs, quel que soit le mode de réalisation, la première propriété optique estimée par la première modalité, en différentes régions d'intérêt de l'échantillon, peut être recalée, dans au moins une région d'intérêt, en utilisant une première propriété optique auxiliaire mesurée par la deuxième modalité.

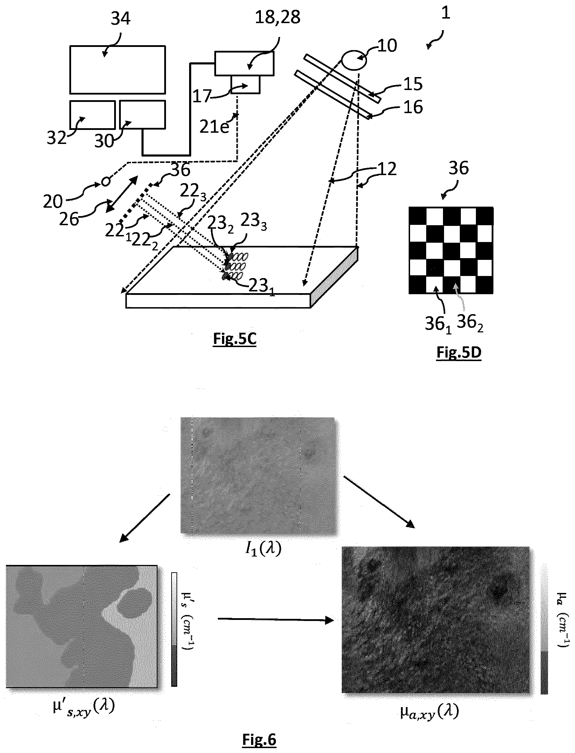

L'avantage de l'imagerie de réflectance est d'obtenir une estimation de la première propriété optique de l'échantillon en différentes zones d'intérêt ROI de la première image $I_1(\lambda)$, chaque région d'intérêt ROI étant définie par un pixel ou une pluralité de pixels (x,y) de la première image. Ainsi, lorsque la première propriété optique est un coefficient d'absorption, on obtient une distribution spatiale $\mu_{a-x,y}(\lambda)$ du coefficient d'absorption à la surface 5s de l'échantillon. La deuxième modalité permet de mesurer une propriété optique auxiliaire de l'échantillon, par exemple un coefficient de diffusion réduit $\mu'_s(\lambda)$ en une ou plusieurs éléments de surface $5_i$ de l'échantillon, comme décrit par la suite, en particulier en lien avec les figures 5B et 5C.

Selon une variante, à l'issue de l'étape 150, l'étape 140 peut être réitérée, en utilisant, dans l'expression (4), un premier facteur de calibration tenant compte de la première propriété optique résultant de l'étape 150 pour la région d'intérêt considérée, et éventuellement de la propriété optique auxiliaire. Un tel facteur de calibration peut être obtenu à partir d'un échantillon de calibration dont la première propriété optique correspond à la première propriété optique résultant de l'étape 150, ou par interpolation entre des facteurs de calibration obtenus respectivement à partir de différents échantillons, l'interpolation visant à estimer un facteur de calibration correspondant à la première propriété optique préalablement estimée, et/ou la propriété optique auxiliaire mesurée sur l'échantillon. Les étapes 150 et 140 peuvent ainsi être réitérées jusqu'à l'atteinte d'un critère de convergence, par exemple un nombre d'itérations prédéterminé ou un faible écart entre deux premières propriétés optiques respectivement estimées au cours de deux itérations successives. Autrement dit, au cours de cette variante, on applique, lors de la première modalité, un premier facteur de calibration adapté à la première propriété optique de l'échantillon, et éventuellement à la propriété optique auxiliaire de l'échantillon. Cela permet d'obtenir une meilleure estimation de la première propriété optique. Précisons qu'un tel procédé itératif ne nécessite pas une mesure de la propriété optique auxiliaire, mais peut se baser sur une estimation de cette dernière, sachant qu'il demeure préférable de procéder à une mesure de la propriété optique auxiliaire sur l'échantillon examiné.

Les inventeurs ont comparé l'estimation du coefficient d'absorption $\mu_a(\lambda)$, par imagerie spectrale de réflectance, en considérant soit une valeur arbitraire d'un coefficient de diffusion réduit, comme dans l'art antérieur, soit une valeur de ce coefficient mesurée par DRS, sur l'échantillon examiné. Ces estimations ont été réalisées selon trois longueurs d'onde, respectivement 500 nm, 600 nm et 700 nm.

La figure 4A représente les estimations de $\mu_a(\lambda)$ des quatre échantillons test 1%IL, 1,5%IL, 2%IL, 3%IL, dont la concentration volumique en intralipide est respectivement de 1%, 1,5%, 2% et 3%. Les estimations sont représentées en fonction de la longueur d'onde. Ces échantillons test présentent un même coefficient d'absorption $\mu_a(\lambda)$, ce dernier étant représenté en pointillés sur les figures 4A et 4C. Sa valeur s'élève à 0.4 cm$^{-1}$ à 600 nm. Chaque échantillon a son propre coefficient de diffusion réduit, de telle sorte que le pourcentage d'intralipide correspond à une valeur d'un coefficient de diffusion réduit. Ces estimations sont effectuées en se basant sur des images de chaque échantillon de test, telles que représentées sur la figure 3A. Sur cette dernière, on a représenté des régions d'intérêt identifiées par un pourcentage, chaque région d'intérêt correspondant à un échantillon test. Chaque région d'intérêt a été traitée en considérant une mesure du coefficient de diffusion réduit $\mu'_s(\lambda)$ effectuée sur l'échantillon de test 1.5%IL, ce dernier étant, dans cet exemple, considéré comme représentatif des autres échantillons test. On constate que seule l'estimation du coefficient d'absorption $\mu_a(\lambda)$ de l'échantillon 1.5%IL est fiable. Les estimations liées aux autres échantillons sont affectées d'une erreur de mesure importante, bien qu'étant basées sur le coefficient de diffusion réduit $\mu'_s(\lambda)$ de l'échantillon de test 1.5%IL, dont les compositions sont pourtant proches des autres échantillons de test.

La figure 4B illustre l'erreur relative moyenne des estimations du coefficient d'absorption $\mu_a(\lambda)$ dans les trois longueurs d'onde, représentées sur la figure 4A, pour chaque échantillon de test.

Seule l'estimation concernant l'échantillon 1.5%IL est satisfaisante.

Afin d'obtenir une estimation fiable du coefficient d'absorption, il est nécessaire de se baser sur une mesure du coefficient de diffusion réduit $\mu'_s(\lambda)$ sur l'échantillon examiné, et non sur d'autres estimations, même lorsque ces dernières sont obtenues à partir d'échantillons dont les compositions comme proches de l'échantillon examiné. La figure 4C représente les estimations de $\mu_a(\lambda)$ obtenues sur chaque échantillon test 1%IL, 1,5%IL, 2%IL, 3%IL en considérant 4 valeurs différentes du coefficient de diffusion réduit $\mu'_s(\lambda)$, ces valeurs ayant été respectivement mesurées sur chacun de ces échantillons de test. Le coefficient de diffusion réduit $\mu'_s(\lambda)$ de chaque échantillon test a été estimé par DRS aux trois longueurs d'onde mentionnées ci-dessus. On a ensuite estimé le coefficient d'absorption $\mu_a(\lambda)$ de chaque échantillon de test par imagerie spectrale de réflectance, en prenant en compte le coefficient de diffusion réduit mesuré sur chacun d'entre eux. On observe que les valeurs estimées de $\mu_a(\lambda)$ sont nettement plus proches de la valeur réelle.

La figure 4D illustre l'erreur moyenne de l'estimation du coefficient d'absorption $\mu_a(\lambda)$, dans les trois longueurs d'onde. On observe que ces erreurs sont moindres que celles représentées sur la figure 4B. La mesure du coefficient d'absorption $\mu_a(\lambda)$ d'un échantillon, basée sur une mesure du coefficient de diffusion réduit $\mu_s'(\lambda)$ mesuré sur ledit échantillon, est donc plus fiable.

Les inventeurs ont réalisé une autre série de mesures en mesurant la propriété optique auxiliaire, c'est-à-dire le coefficient de diffusion réduit, de façon itérative, le deuxième facteur de calibration prenant en compte ledit coefficient mesuré lors d'une itération précédente. Cette méthode d'adaptation du facteur de calibration, précédemment décrite, permet d'adapter le deuxième facteur de calibration aux propriétés optiques de l'échantillon, et résulte en une meilleure estimation du coefficient de diffusion réduit. De ce fait, l'erreur de mesure de la première propriété optique, c'est-à-dire le coefficient d'absorption, est systématiquement inférieure à 5%. La précision de la mesure est donc améliorée.

Un des points clefs de l'approche proposée par les inventeurs est d'effectuer une mesure locale d'une propriété optique auxiliaire afin de mieux estimer une première propriété optique à partir de la première image $I_1(\lambda)$. La propriété optique auxiliaire est estimée ponctuellement, au niveau d'un élément de surface 5i de la surface 5s de l'échantillon. La localisation de cet élément de surface 5i peut être déterminée à partir de la première image $I_1(\lambda)$. Cette première image peut par exemple être segmentée en zones présentant des pixels $I_{1\,(x,y)}$ de même intensité, ou d'intensité comparable, la propriété optique auxiliaire étant estimée sur une ou plusieurs zones issues de cette segmentation. Ainsi, la première image $I_1(\lambda)$ peut permettre de définir la localisation de l'élément de surface 5i ou un maillage d'éléments de surface 5i sur lesquels la propriété optique auxiliaire est mesurée. La propriété optique auxiliaire peut être mesurée successivement, sur chaque élément de surface ainsi défini, en appliquant les étapes 200 à 240 précédemment décrites, le dispositif représenté sur la figure 1B effectuant un balayage à la surface de l'échantillon.

Selon une variante, les éléments de surface 5i sont déterminés arbitrairement, par exemple sous la forme d'un maillage, régulier ou non, établi à la surface de l'échantillon. La propriété optique auxiliaire est alors mesurée en chaque point de ce maillage.

L'application de la deuxième modalité de mesure sur différents éléments de surface 5i permet une mesure discrète de la propriété optique auxiliaire, selon chaque élément de surface réparti discrètement à la surface de l'échantillon, selon un maillage. Il est alors possible d'interpoler les différentes mesures, de façon à estimer la propriété optique auxiliaire entre chaque maille. L'interpolation peut être linéaire ou polynomiale. Ainsi, la première propriété optique, au niveau d'une région d'intérêt ROI de la première image $I_1(\lambda)$ peut être déterminée à partir d'une propriété optique auxiliaire, soit mesurée, au niveau cette région d'intérêt, lorsque cette dernière coïncide avec un élément de surface 5i, ou lorsque l'élément de surface 5i est inclus dans la région d'intérêt, soit interpolée à partir de propriétés optiques auxiliaires respectivement mesurées en différents éléments de surface de l'échantillon, en particulier adjacents de ladite région d'intérêt.

Les figures 5A à 5C représentent des variantes du dispositif 1. Sur la figure 5A, on a représenté la deuxième composante 1b permettant d'effectuer des mesures par DRS. Sur cette figure, par rapport à la figure 1B, on a supprimé les fibres optiques de détection 27 ainsi que le commutateur optique 29. Le photodétecteur auxiliaire 28 est un capteur d'image auxiliaire, ou deuxième capteur d'image. Selon cette configuration, le photodétecteur auxiliaire n'est pas spectralement résolu, la bande spectrale du faisceau lumineux auxiliaire 22 étant modulable. La source de lumière auxiliaire 20 peut être associée à des filtres optiques, par exemple sous la forme d'une roue à filtre 35. De façon alternative, la source de lumière auxiliaire comporte des sources élémentaires de lumières, par exemple des diodes laser ou des diodes électroluminescentes, émettant dans différentes bandes spectrales. Le capteur d'image auxiliaire 28 est optiquement couplé à la surface 5s de l'échantillon, de telle sorte que les zones élémentaires de rétrodiffusion 25 soient distinctes les unes des autres, et distinctes de la zone élémentaire d'illumination 23. La source de lumière auxiliaire peut émettre un faisceau lumineux auxiliaire collimaté, formant la zone d'illumination 23, auquel cas la fibre optique d'illumination 21 et son optique 26 peut être supprimée. De même que dans la figure 1B, une fibre de retour d'excitation 21e peut guider le faisceau lumineux auxiliaire 22 émis vers le photodétecteur auxiliaire 28.

La figure 5B représente un dispositif 1 comportant les premières et deuxièmes composantes la et 1b. Sur ce dispositif, le premier capteur d'image 18 et le capteur d'image auxiliaire 28 sont confondus et forment un même capteur d'image, permettant d'acquérir :

- une première image $I_1(\lambda)$ de l'échantillon 5 lorsque ce dernier est illuminé par un premier faisceau lumineux 12, ce dernier couvrant une surface étendue de l'échantillon ;
- une deuxième image $I_2(\lambda)$ de l'échantillon 5 lorsque ce dernier est illuminé par un faisceau lumineux auxiliaire 22, ce dernier formant une zone élémentaire d'illumination 23 sur la surface de l'échantillon.

Le premier faisceau 12 et le faisceau auxiliaire 22 sont respectivement émis par la première source de lumière 10 et par la source de lumière auxiliaire 20 dans une bande spectrale, et éventuellement successivement dans différentes bandes spectrales. L'optique 17 peut être adaptée entre l'acquisition de la première image et de la deuxième image. De même que dans les figures 1B et 5A, une fibre de retour d'excitation 21e peut guider le faisceau lumineux auxiliaire 22 émis vers le photodétecteur auxiliaire 28.

La première image $I_1(\lambda)$ est traitée par le microprocesseur 30, de façon à estimer une première propriété optique, selon les étapes 100 à 150 préalablement décrites. La deuxième image $I_2(\lambda)$ est traitée par le microprocesseur 30 de façon à déterminer, sur cette image, l'intensité de signaux rétrodiffusés $S_n(\lambda)$ à différentes distances de rétrodiffusion $D_n$ puis à mettre en œuvre le procédé décrit en lien avec les étapes 200 à 240 pour mesurer une propriété optique auxiliaire de l'échantillon, cette dernière étant utilisée, lors de l'étape 150, pour estimer la première propriété optique.

Dans les modes de réalisation précédemment décrits, la première image $I_1(\lambda)$ permet une estimation d'une propriété optique à partir de différentes régions d'intérêt ROI de la première image, chaque région d'intérêt correspondant à un pixel $I_{1,xy}(\lambda)$, ou à un groupe de pixels, de ladite image. La deuxième modalité permet d'estimer ponctuellement, c'est-à-dire au niveau d'un élément de surface 5i, la propriété optique auxiliaire. Le faisceau lumineux auxiliaire 22 peut être successivement dirigé vers différents éléments de surface 5i, de façon à estimer successivement la propriété optique auxiliaire de l'échantillon au niveau de chaque élément de surface 5i.

Le mode de réalisation présenté sur la figure 5C permet une formation simultanée de différentes zones élémentaires d'illumination 23 réparties à la surface de l'échantillon. Pour cela, on a disposé un masque 36 entre la source de lumière auxiliaire 20 et la surface de l'échantillon 5. Un tel masque est constitué de portions transparentes $36_1$ et de portions opaques $36_2$, ces portions étant projetées à la surface de l'échantillon 5. La figure 5D montre un exemple d'un tel masque. On obtient alors autant de zones d'illumination élémentaires $23_1, 23_2, 23_3$ que de portions transparentes projetées. On peut disposer un système optique 26 pour focaliser le faisceau lumineux auxiliaire 22 sur la surface de l'échantillon. Une fibre de retour d'excitation 21e peut relier la source de lumière auxiliaire 20 au capteur d'image. Le capteur d'image auxiliaire 28, qui est ici confondu avec le premier capteur d'image 18, forme une deuxième image $I_2(\lambda)$, sur laquelle apparaissent différentes zones élémentaires d'illumination 23 et, les différentes zones de rétrodiffusion élémentaires 25 associées à chaque zone élémentaire d'illumination. Les étapes 200 à 240 sont mises en œuvre à partir de l'intensité des zones de rétrodiffusion élémentaires 25 associées à une même zone élémentaire d'illumination 23. La propriété optique auxiliaire est estimée, en différentes localisations discrètes 5i, à partir de la même image $I_2(\lambda)$. Ce dispositif évite un balayage du faisceau lumineux auxiliaire 20 à la surface de l'échantillon, puisque le masque permet une formation simultanée de plusieurs faisceaux auxiliaires $22_1, 22_2, 22_3...$ Le masque peut être constitué d'un arrangement régulier de portions opaques et de portions transparentes. Il peut s'agir d'une grille ou d'une plaque de verre sur laquelle un motif opaque a été formé. Il peut également s'agir d'un écran à cristaux liquides, ce qui permet de former un masque dont le motif opaque est modulable.

La figure 6 schématise un mode de réalisation de l'invention, appliqué sur la surface d'un échantillon. Une première image $I_1(\lambda)$ est formée, à partir de laquelle on détermine des éléments de surface sur lesquels on mesure, de façon discrète, la propriété optique auxiliaire, en l'occurrence le coefficient de diffusion réduit. L'interpolation des mesures permet d'obtenir une répartition spatiale $\mu'_{s,xy}(\lambda)$ de la propriété optique auxiliaire $\mu_s'(\lambda)$. Cette répartition spatiale est utilisée pour estimer le coefficient d'absorption $\mu_a(\lambda)$, et d'obtenir une répartition spatiale $\mu_{a,xy}(\lambda)$ de ce dernier. On dispose alors d'une estimation quantitative de la distribution spatiale des propriétés optiques d'absorption et de diffusion à la surface de l'échantillon examiné.

La méthode et le dispositif objets de l'invention pourront être mis en œuvre sur des échantillons biologiques, par exemple des tissus biologiques, à des fins d'aide au diagnostic, notamment dans le cas de pathologies de la peau. Elle peut également être mise en œuvre dans le suivi de la vascularisation de la peau, par exemple suite à des greffes, ou à l'analyse d'échantillons de peau prélevés au cours d'une opération, en particulier pour délimiter des zones tumorales de zones saines.

Au-delà des échantillons biologiques, la méthode pourra être mise en œuvre pour contrôler des échantillons, par exemple dans l'industrie agroalimentaire ou le contrôle de l'environnement.

## Revendications

1. Procédé d'estimation de propriétés optiques d'un échantillon, chaque propriété optique gouvernant la diffusion ou l'absorption de la lumière dans l'échantillon, le procédé comportant les étapes suivantes :

   a) illumination de la surface de l'échantillon à l'aide d'un premier faisceau lumineux (12), produit par une première source de lumière (10) ;
   b) acquisition, à travers un objectif (17), et à l'aide d'un premier capteur d'image (18), d'une première image ($I_1(\lambda)$) d'un rayonnement lumineux (14) rétrodiffusé par l'échantillon ainsi illuminé ;
   c) à l'aide de ladite première image, détermination, en plusieurs régions d'intérêts (*ROI*) de la surface de l'échantillon (5s), d'une première grandeur d'intérêt ($R(\lambda)$), représentative d'une quantité dudit rayonnement lumineux rétrodiffusé (14) par l'échantillon ;
   d) estimation d'une première propriété optique ($\mu_a(\lambda)$) en chacune desdites régions d'intérêt (*ROI*), à l'aide de la première grandeur d'intérêt ;
   le procédé étant **caractérisé en ce qu'**il comporte, préalablement à l'étape d),

   - une détermination d'au moins un élément de surface (5i) de l'échantillon à partir de la première image ($I_1(\lambda)$) acquise lors de l'étape b) ;
   - une mesure d'une propriété optique dite auxiliaire ($\mu'_s(\lambda)$) de l'échantillon, différente de la première propriété optique, la propriété optique auxiliaire étant mesurée ponctuellement selon chaque élément de surface (5i) ainsi déterminé ;

   de telle sorte que chaque estimation réalisée lors de l'étape d) prend en compte une propriété optique auxiliaire ainsi mesurée sur l'échantillon ;
   le procédé étant tel que :

   - soit la première propriété optique est une propriété d'absorption de la lumière ($\mu_a(\lambda)$) de l'échantillon, la propriété optique auxiliaire étant une propriété de diffusion de la lumière ($\mu'_s(\lambda)$) dans l'échantillon ;
   - soit la première propriété optique est une propriété de diffusion de la lumière ($\mu'_s(\lambda)$) de l'échantillon, la propriété optique auxiliaire étant une propriété d'absorption de la lumière ($\mu_a(\lambda)$) dans l'échantillon.

2. Procédé selon l'une quelconque des revendications précédentes, dans lequel l'étape de mesure de la propriété optique auxiliaire ($\mu'_s(\lambda)$) comporte les sous-étapes suivantes :

   i) illumination de la surface de l'échantillon à l'aide d'un faisceau lumineux auxiliaire (22), produit par une source de lumière auxiliaire (20), de façon à former, à la surface de l'échantillon, une zone élémentaire d'illumination (23), correspondant à la partie de la surface illuminée par ledit faisceau de lumineux auxiliaire ;
   ii) mesure, à l'aide d'un photodétecteur auxiliaire (28), d'un signal de rétrodiffusion ($S_n(\lambda)$), représentatif d'un rayonnement rétrodiffusé (24) par l'échantillon, ledit rayonnement rétrodiffusé émanant d'une zone élémentaire de rétrodiffusion (25), séparée de la zone élémentaire d'illumination (23) et située à une distance ($D_n$), dite distance de rétrodiffusion, de cette dernière ;
   iii) estimation de la propriété optique auxiliaire de l'échantillon en fonction du signal de rétrodiffusion ($S_n(\lambda)$)

mesuré lors de la sous-étape ii).

3. Procédé selon la revendication précédente dans lequel :

- la sous-étape ii) comporte l'obtention, à partir du signal de rétrodiffusion ($S_n(\lambda)$), d'une grandeur d'intérêt dite auxiliaire ($R_n(\lambda)$), représentative d'une quantité du rayonnement (24) rétrodiffusé par l'échantillon, à une distance de rétrodiffusion ($D_n$), suite à son illumination par le faisceau lumineux auxiliaire;
- la sous-étape iii) comporte une comparaison de ladite grandeur d'intérêt auxiliaire ($R_n(\lambda)$) avec une pluralité d'estimations $\left(R_{n,\mu a,\mu s'}^{model}(\lambda)\right)$ de cette grandeur d'intérêt, chaque estimation étant réalisée en considérant une valeur prédéterminée de ladite propriété optique auxiliaire ($\mu'_s(\lambda)$).

4. Procédé selon l'une quelconque des revendications 2 ou 3 dans lequel :

- lors de la sous-étape i), l'échantillon est illuminé successivement ou simultanément par une pluralité de faisceaux lumineux auxiliaires ($22_1$, $22_2$, $22_3$) de façon à former, à la surface de ce dernier, une pluralité de zones élémentaires d'illumination ($23_1$, $23_2$, $23_3$) espacées les unes des autres ;
- les sous-étapes ii) et iii) sont mises en œuvre en considérant un signal rétrodiffusé émis à une distance de rétrodiffusion ($D_n$) de chaque zone élémentaire d'illumination, de façon à estimer la propriété optique auxiliaire dans autant d'éléments de surface (5i) que de faisceaux lumineux auxiliaires.

5. Procédé selon l'une quelconque des revendications précédentes, dans lequel l'étape d) comporte, dans chaque région d'intérêt (ROI), une comparaison de la première grandeur d'intérêt ($R(\lambda)$) déterminée lors de l'étape c) avec une pluralité d'estimations $\left(R_{(\mu a,\mu'_s)}^{model}(\lambda)\right)$ de cette grandeur d'intérêt, chaque estimation étant réalisée en considérant une valeur prédéterminée de la première propriété optique ($\mu_a(\lambda)$), et en prenant en compte la propriété optique auxiliaire ($\mu'_s(\lambda)$) préalablement mesurée sur l'échantillon.

6. Procédé selon l'une quelconque des revendications précédentes, dans lequel la propriété optique auxiliaire ($\mu'_s(\lambda)$) de l'échantillon est mesurée en une pluralité d'éléments de surface (5i) de l'échantillon.

7. Procédé selon la revendication 6, comportant une interpolation de la propriété optique auxiliaire ($\mu'_s(\lambda)$) à partir d'au moins deux propriétés optiques auxiliaires préalablement mesurées, de façon à établir une distribution spatiale ($\mu'_{s,xy}(\lambda)$) de ladite propriété optique auxiliaire.

8. Dispositif (1) pour estimer des propriétés optiques d'un échantillon (5), chaque propriété optique gouvernant la diffusion ou l'absorption de la lumière dans l'échantillon, le dispositif comportant :

- une première source de lumière (10), apte à émettre un premier faisceau lumineux (12) se propageant vers l'échantillon ;
- un premier capteur d'image (18), apte à acquérir une première image ($I_1(\lambda)$), à travers un objectif (17), d'un premier rayonnement lumineux (14) rétrodiffusé par l'échantillon ;
- une source de lumière auxiliaire (20), apte à émettre un faisceau lumineux auxiliaire (22) se propageant vers l'échantillon, de façon à former, à la surface de ce dernier, une zone élémentaire d'illumination (23), correspondant à la partie d'une surface (5s) de l'échantillon illuminée par ledit faisceau lumineux auxiliaire ;
- un photodétecteur auxiliaire (28) apte à mesurer un rayonnement rétrodiffusé (24) par l'échantillon, à une distance de rétrodiffusion ($D_n$) de la zone élémentaire d'illumination (23), suite à l'illumination de l'échantillon par la source de lumière auxiliaire (20) ;
- un microprocesseur (30), configuré pour déterminer une propriété optique auxiliaire ($\mu'_s(\lambda)$) de l'échantillon à partir du rayonnement rétrodiffusé (24) mesuré par le photodétecteur auxiliaire (28), le microprocesseur (30) étant également configuré pour déterminer une première propriété optique ($\mu_a(\lambda)$) différente de la propriété optique auxiliaire, en différents points de la surface (5s) de l'échantillon, à partir de la première image acquise par le premier capteur d'image (18), en prenant en compte la propriété optique auxiliaire préalablement déterminée, la première propriété optique et la propriété optique auxiliaire étant respectivement :

- soit une propriété d'absorption de la lumière ($\mu_a(\lambda)$) de l'échantillon, et une propriété de diffusion de la lumière ($\mu'_s(\lambda)$) de l'échantillon ;

- soit une propriété de diffusion de la lumière ($\mu'_s(\lambda)$) de l'échantillon et une propriété d'absorption de la lumière ($\mu_a(\lambda)$) de l'échantillon.

9. Dispositif selon la revendication 8, dans lequel le photodétecteur auxiliaire (28) est un capteur d'image.

10. Dispositif selon la revendication 9, dans lequel le photodétecteur auxiliaire (28) est le premier capteur d'image (18).

11. Dispositif selon l'une quelconque des revendications 8 à 10, dans lequel la source de lumière auxiliaire (20) est apte à former simultanément une pluralité de zones élémentaires d'illumination ($23_1$, $23_2$, $23_3$), espacées les unes des autres, à la surface de l'échantillon (5).

12. Dispositif selon la revendication 11, comportant un masque (36), interposé entre la source de lumière auxiliaire (20) et l'échantillon, le masque comportant des portions transparentes (36a), permettant le passage du faisceau lumineux auxiliaire (22), et des portions opaques (36b), bloquant le faisceau lumineux auxiliaire, de telle sorte que chaque zone élémentaire d'illumination ($23_1$, $23_2$, $23_3$) est formée par une projection d'une portion transparente (36a) sur la surface de l'échantillon.

13. Dispositif selon l'une quelconque des revendications 10 à 12, dans lequel le microprocesseur est apte à mettre en œuvre les étapes c) et d) du procédé objet de l'une quelconque des revendications 1 à 8.

**Patentansprüche**

1. Verfahren zur Schätzung optischer Eigenschaften einer Probe, wobei jede optische Eigenschaft die Streuung oder Absorption des Lichts in der Probe steuert, wobei das Verfahren die folgenden Schritte aufweist:

   a) Beleuchten der Oberfläche der Probe mithilfe eines ersten Lichtbündels (12), das von einer ersten Lichtquelle (10) erzeugt wird;
   b) Erfassen, durch ein Objektiv (17) und mithilfe eines ersten Bildsensors (18), eines ersten Bilds ($I_1(\lambda)$) einer Lichtstrahlung (14), die von der so beleuchteten Probe rückgestreut wird;
   c) mithilfe des ersten Bilds, Bestimmen, in mehreren Interessensgebieten (ROI) der Oberfläche der Probe (5s), einer ersten Interessensgröße (R(A)), die für eine Menge der von der Probe rückgestreuten Lichtstrahlung (14) repräsentativ ist;
   d) Schätzen einer ersten optischen Eigenschaft ($\mu_a(\lambda)$) in jedem der Interessensgebiete (ROI), mithilfe der ersten Interessensgröße;
   wobei das Verfahren **dadurch gekennzeichnet ist, dass** vor dem Schritt d) Folgendes aufweist,

   - ein Bestimmen wenigstens eines Oberflächenelements (5i) der Probe anhand des ersten Bilds ($I_1(\lambda)$), das beim Schritt b) erfasst wird;
   - ein Messen einer optischen Hilfseigenschaft ($\mu'_s(\lambda)$) der Probe, die von der ersten optischen Eigenschaft verschieden ist, wobei die optische Hilfseigenschaft punktuell gemäß jedem so bestimmten Oberflächenelement (5i) gemessen wird;

   so dass jede Schätzung, die beim Schritt d) durchgeführt wird, eine optische Hilfseigenschaft berücksichtigt, die so auf der Probe gemessen wird;
   wobei das Verfahren so geartet ist, dass:

   - entweder die erste optische Eigenschaft eine Absorptionseigenschaft des Lichts ($\mu_a(\lambda)$) der Probe ist, wobei die optische Hilfseigenschaft eine Streuungseigenschaft des Lichts ($\mu's(\lambda)$) in der Probe ist;
   - oder die erste optische Eigenschaft eine Streuungseigenschaft des Lichts ($\mu's(\lambda)$) der Probe ist, wobei die optische Hilfseigenschaft eine Absorptionseigenschaft des Lichts ($\mu_a(\lambda)$) in der Probe ist.

2. Verfahren nach einem der vorhergehenden Ansprüche, wobei der Schritt des Messens der optischen Hilfseigenschaft ($\mu's(\lambda)$) die folgenden Unterschritte aufweist:

   i) Beleuchten der Oberfläche der Probe mithilfe eines Hilfslichtbündels (22), das von einer Hilfslichtquelle (20) erzeugt wird, so dass an der Oberfläche der Probe ein elementarer Beleuchtungsbereich (23) gebildet wird, der dem Abschnitt der Oberfläche entspricht, der vom Hilfslichtbündel beleuchtet wird;

ii) Messen, mithilfe eines Hilfsphotodetektors (28), eines Rückstreusignals ($S_n(\lambda)$), das für eine von der Probe rückgestreute Strahlung (24) repräsentativ ist, wobei die rückgestreute Strahlung von einem elementaren Rückstreubereich (25) ausgeht, der von dem elementaren Beleuchtungsbereich (23) getrennt ist und sich in einem Abstand ($D_n$) von letzterem befindet, der Rückstreuabstand genannt wird;

iii) Schätzen der optischen Hilfseigenschaft der Probe in Abhängigkeit von dem Rückstreusignal ($S_n(\lambda)$), das beim Unterschritt ii) gemessen wird.

3. Verfahren nach dem vorhergehenden Anspruch, wobei:

- der Unterschritt ii) das Erhalten einer Hilfsinteressensgröße ($R_n(\lambda)$) anhand des Rückstreusignals ($S_n(\lambda)$) aufweist, die für eine Menge der Strahlung (24) repräsentativ ist, die von der Probe in einem Rückstreuabstand ($D_n$) rückgestreut wird, nach deren Beleuchtung durch das Hilfslichtbündel;
- der Unterschritt iii) ein Vergleichen der Hilfsinteressensgröße ($R_n(\lambda)$) mit einer Mehrzahl von Schätzungen

$$\left( R^{model}_{(n,\mu\alpha,\mu s')}(\lambda) \right)$$

dieser Interessensgröße aufweist, wobei jedes Schätzen unter Berücksichtigung eines vorbestimmten Werts der optischen Hilfseigenschaft ($\mu$'s($\lambda$)) durchgeführt wird.

4. Verfahren nach einem der Ansprüche 2 oder 3, wobei:

- beim Unterschritt i) die Probe nacheinander oder gleichzeitig von einer Mehrzahl von Hilfslichtbündeln ($22_1$, $22_2$, $22_3$) so beleuchtet wird, dass an der Oberfläche derselben eine Mehrzahl von elementaren Beleuchtungsbereichen ($23_1$, $23_2$, $23_3$) gebildet wird, die voneinander beabstandet sind;
- die Unterschritte ii) und iii) unter Berücksichtigung eines rückgestreuten Signals durchgeführt werden, das in einem Rückstreuabstand ($D_n$) von jedem elementaren Beleuchtungsbereich gesendet wird, um die optische Hilfseigenschaft in so vielen Oberflächenelementen (5i) wie Hilfslichtbündeln zu schätzen.

5. Verfahren nach einem der vorhergehenden Ansprüche, wobei der Schritt d) in jedem Interessensgebiet (ROI) ein Vergleichen der ersten Interessensgröße (R(A)), die beim Schritt c) bestimmt wird,

mit einer Mehrzahl von Schätzungen $(R^{model}_{(\mu\alpha,\mu's)}(\lambda))$ dieser Interessensgröße aufweist, wobei jede Schätzung unter Berücksichtigung eines vorbestimmten Werts der ersten optischen Eigenschaft ($\mu_a(\lambda)$) und unter Berücksichtigung der optischen Hilfseigenschaft ($\mu$'s($\lambda$)) durchgeführt wird, die zuvor an der Probe gemessen wurde.

6. Verfahren nach einem der vorhergehenden Ansprüche, wobei die optische Hilfseigenschaft ($\mu$'s($\lambda$)) der Probe in einer Mehrzahl von Oberflächenelementen (5i) der Probe gemessen wird.

7. Verfahren nach Anspruch 6, aufweisend eine Interpolation der optischen Hilfseigenschaft ($\mu$'s($\lambda$)) anhand von wenigstens zwei zuvor gemessenen optischen Hilfseigenschaften, um eine räumliche Verteilung ($\mu'_{S,xy}(\lambda)$) der optischen Hilfseigenschaft herzustellen.

8. Vorrichtung (1) zum Schätzen optischer Eigenschaften einer Probe (5), wobei jede optische Eigenschaft die Streuung oder Absorption des Lichts in der Probe steuert, die Vorrichtung aufweisend:

- eine erste Lichtquelle (10), die geeignet ist, ein erstes Lichtbündel (12) zu emittieren, das sich zur Probe hin ausbreitet;
- einen ersten Bildsensor (18), der geeignet ist, ein erstes Bild ($I_1(\lambda)$) einer ersten Lichtstrahlung (14), die von der Probe rückgestreut wird, durch ein Objektiv (17) zu erfassen;
- eine Hilfslichtquelle (20), die geeignet ist, ein Hilfslichtbündel (22) zu emittieren, das sich zur Probe hin ausbreitet, so dass an der Oberfläche derselben ein elementarer Beleuchtungsbereich (23) gebildet wird, der dem Abschnitt einer Oberfläche (5s) der Probe entspricht, der vom Hilfslichtbündel beleuchtet wird;
- einen Hilfsphotodetektor (28), der geeignet ist, eine von der Probe rückgestreute Strahlung (24) in einem Rückstreuabstand ($D_n$) vom elementaren Beleuchtungsbereich (23) nach der Beleuchtung der Probe durch die Hilfslichtquelle (20) zu messen;
- einen Mikroprozessor (30), der dazu ausgebildet ist, eine optische Hilfseigenschaft ($\mu$'s($\lambda$)) der Probe anhand der rückgestreuten Strahlung (24) zu bestimmen, die vom Hilfsphotodetektor (28) gemessen wird, wobei der Mikroprozessor (30) ebenfalls dazu ausgebildet ist, eine erste optische Eigenschaft ($\mu_a(\lambda)$), die von der optischen Hilfseigenschaft verschieden ist, an verschiedenen Punkten der Oberfläche (5s) der Probe anhand des ersten Bilds zu bestimmen, das vom ersten Bildsensor (18) erfasst wird, unter Berücksichtigung der zuvor bestimmten optischen Hilfseigenschaft, wobei die erste optischer Eigenschaft und die optische Hilfseigenschaft jeweils Folgendes sind:

- entweder eine Absorptionseigenschaft des Lichts ($\mu_a(\lambda)$) der Probe und eine Streuungseigenschaft des Lichts ($\mu's(\lambda)$) der Probe;
- oder eine Streuungseigenschaft des Lichts ($\mu's(\lambda)$) der Probe und eine Absorptionseigenschaft des Lichts ($\mu_a(\lambda)$) der Probe.

9. Vorrichtung nach Anspruch 8, wobei der Hilfsphotodetektor (28) ein Bildsensor ist.

10. Vorrichtung nach Anspruch 9, wobei der Hilfsphotodetektor (28) der erste Bildsensor (18) ist.

11. Vorrichtung nach einem der Ansprüche 8 bis 10, wobei die Hilfslichtquelle (20) geeignet ist, gleichzeitig eine Mehrzahl von voneinander beabstandeten elementaren Beleuchtungsbereichen ($23_1$, $23_2$, $23_3$) an der Oberfläche der Probe (5) zu bilden.

12. Vorrichtung nach Anspruch 11, aufweisend eine Maske (36), die zwischen der Hilfslichtquelle (20) und der Probe angeordnet ist, wobei die Maske transparente Teilbereiche (36a), die den Durchtritt des Hilfslichtbündels (22) ermöglichen, und lichtundurchlässige Abschnitte (36b), die das Hilfslichtbündel blockieren, aufweist, so dass jeder elementare Beleuchtungsbereich ($23_1$, $23_2$, $23_3$) von einer Projektion eines transparenten Teilbereichs (36a) auf der Oberfläche der Probe gebildet ist.

13. Vorrichtung nach einem der Ansprüche 10 bis 12, wobei der Mikroprozessor geeignet ist, die Schritte c) und d) des Verfahrens nach einem der Ansprüche 1 bis 8 durchzuführen.


**Claims**

1. Method for estimating optical properties of a sample, each optical property governing the scattering or the absorption of light in the sample, the method including the following steps:

    a) illuminating the surface of the sample using a first light beam (12), produced by a first light source (10);
    b) acquiring, through an objective (17), and using a first image sensor (18), a first image ($I_1(\lambda)$) of light radiation (14) backscattered by the sample thus illuminated;
    c) using said first image, determining, at multiple regions of interest (ROI) of the surface (5s) of the sample, a first quantity of interest (R(A)), representative of a quantity of said light radiation (14) backscattered by the sample;
    d) estimating a first optical property ($\mu_a(\lambda)$) at each of said regions of interest (ROI), using the first quantity of interest;
    the method being **characterized in that** it comprises, prior to step d),

        - determining at least one surface element (5i) of the sample on the basis of the first image ($I_1(\lambda)$) acquired in step b);
        - measuring an optical property, referred to as the auxiliary optical property ($\mu'_s(\lambda)$) of the sample, that differs from the first optical property, the auxiliary optical property being measured in a point-like manner according to each surface element (5i) thus determined;

    such that each estimate made in step d) takes account of an auxiliary optical property thus measured on the sample;
    the method being such that:

        - either the first optical property is a light absorption property ($\mu_a(\lambda)$) of the sample, the auxiliary optical property being a light scattering property ($\mu'_s(\lambda)$) of the sample;
        - or the first optical property is a light scattering property ($\mu'_s(\lambda)$) of the sample, the auxiliary optical property being a light absorption property ($\mu_a(\lambda)$) of the sample.

2. Method according to any one of the preceding claims, in which the step of measuring the auxiliary optical property ($\mu'_s(\lambda)$) includes the following sub-steps:

i) illuminating the surface of the sample using an auxiliary light beam (22), produced by an auxiliary light source (20), so as to form, on the surface of the sample, an elementary illumination zone (23), corresponding to the portion of the surface that is illuminated by said auxiliary light beam;

ii) measuring, using an auxiliary photodetector (28), a backscattering signal ($S_n(\lambda)$), representative of radiation (24) backscattered by the sample, said backscattered radiation emanating from an elementary backscattering zone (25), separated from the elementary illumination zone (23) and located at a distance ($D_n$), referred to as the backscattering distance, from the latter;

iii) estimating the auxiliary optical property of the sample on the basis of the backscattering signal ($S_n(\lambda)$) measured in sub-step ii).

3.  Method according to the preceding claim, in which:

- sub-step ii) includes obtaining, on the basis of the backscattering signal ($S_n(\lambda)$), a quantity of interest, referred to as the auxiliary quantity of interest ($R_n(\lambda)$), representative of a quantity of radiation (24) backscattered by the sample, at a backscattering distance ($D_n$), subsequent to its illumination by the auxiliary light beam;

- sub-step iii) includes comparing said auxiliary quantity of interest ($R_n(\lambda)$) with a plurality of estimates

$$\left(R_{n,\mu a,\mu s'}^{model}(\lambda)\right)$$ of this quantity of interest, each estimate being made by considering a predetermined value of said auxiliary optical property ($\mu'_s(\lambda)$).

4.  Method according to either one of Claims 2 and 3, in which:

- in sub-step i), the sample is successively or simultaneously illuminated by a plurality of auxiliary light beams ($22_1$, $22_2$, $22_3$) so as to form, on the surface of the latter, a plurality of elementary illumination zones ($23_1$, $23_2$, $23_3$) that are spaced apart from one another;

- sub-steps ii) and iii) are implemented by considering a backscattered signal emitted at a backscattering distance ($D_n$) from each elementary illumination zone, so as to estimate the auxiliary optical property in as many surface elements (5i) as there are auxiliary light beams.

5.  Method according to any one of the preceding claims, in which step d) includes, in each region of interest (ROI), comparing the first quantity of interest (R(A)) determined in step c) with a plurality of estimates $\left(R_{(\mu a,\mu' s)}^{model}(\lambda)\right)$ of this quantity of interest, each estimate being made by considering a predetermined value of the first optical property ($\mu_a(\lambda)$), and by taking account of the auxiliary optical property ($\mu'_s(\lambda)$) measured beforehand on the sample.

6.  Method according to any one of the preceding claims, in which the auxiliary optical property ($\mu'_s(\lambda)$) of the sample is measured at a plurality of surface elements (5i) of the sample.

7.  Method according to Claim 6, including interpolating the auxiliary optical property ($\mu'_s(\lambda)$) on the basis of at least two auxiliary optical properties measured beforehand, so as to establish a spatial distribution ($\mu'_{s,xy}(\lambda)$) of said auxiliary optical property.

8.  Device (1) for estimating optical properties of a sample (5), each optical property governing the scattering or the absorption of light in the sample, the device including:

- a first light source (10), capable of emitting a first light beam (12) that is propagated in the direction of the sample;

- a first image sensor (18), capable of acquiring a first image ($I_1(\lambda)$), through an objective (17), of first light radiation (14) backscattered by the sample;

- an auxiliary light source (20), capable of emitting an auxiliary light beam (22) that is propagated in the direction of the sample, so as to form, on the surface of the latter, an elementary illumination zone (23), corresponding to the portion of a surface (5s) of the sample that is illuminated by said auxiliary light beam;

- an auxiliary photodetector (28) capable of measuring radiation (24) backscattered by the sample, at a backscattering distance ($D_n$) from the elementary illumination zone (23), subsequent to the illumination of the sample by the auxiliary light source (20);

- a microprocessor (30), configured to determine an auxiliary optical property ($\mu'_s(\lambda)$) of the sample on the basis of the backscattered radiation (24) measured by the auxiliary photodetector (28), the microprocessor (30) also being configured to determine a first optical property ($\mu_a(\lambda)$) that differs from the auxiliary optical property, at various points on the surface (5s) of the sample, on the basis of the first image acquired by the first image sensor (18), by taking account of the auxiliary optical property determined beforehand, the first optical property and the auxiliary optical property being, respectively:

- either a light absorption property ($\mu_a(\lambda)$) of the sample, and a light scattering property ($\mu'_s(\lambda)$) of the sample;
- or a light scattering property ($\mu'_s(\lambda)$) of the sample and a light absorption property ($\mu_a(\lambda)$) of the sample.

9. Device according to Claim 8, in which the auxiliary photodetector (28) is an image sensor.

10. Device according to Claim 9, in which the auxiliary photodetector (28) is the first image sensor (18).

11. Device according to any one of Claims 8 to 10, in which the auxiliary light source (20) is capable of simultaneously forming a plurality of elementary illumination zones ($23_1$, $23_2$, $23_3$), that are spaced apart from one another, on the surface of the sample (5).

12. Device according to Claim 11, including a mask (36), interposed between the auxiliary light source (20) and the sample, the mask including transparent portions (36a), allowing the auxiliary light beam (22) to pass through, and opaque portions (36b), blocking the auxiliary light beam, such that each elementary illumination zone ($23_1$, $23_2$, $23_3$) is formed by a projection of a transparent portion (36a) onto the surface of the sample.

13. Device according to any one of Claims 10 to 12, in which the microprocessor is capable of implementing steps c) and d) of the method that is the subject of any one of Claims 1 to 8.

34

18

17

10

15

16

$\Omega$

32    30

14

12

5s

5

**Fig.1A**

5i

$1_a$

$1_b$

$21_e$ 29    28

20

$21_p$

34

30    32

21

$27_1$
$27_2$  27
$27_3$

$27_p$

$21_d$

$27_d$

22

26

$24_1$
$24_2$  24
$24_3$

D₃

D₂

5i

$25_1$    $25_3$

5s

D₁

5

$25_2$

23    **Fig.1B**

**Fig. 2**

**Fig. 3A**

**Fig. 3B**

**Fig. 3C**

**Fig. 4A**   λ (nm)

**Fig. 4B**

**Fig. 4C**   λ (nm)

**Fig. 4D**

**Fig.5A**

**Fig.5B**

**Fig.5C**

**Fig.5D**

$I_1(\lambda)$

$\mu'_s \ (cm^{-1})$

$\mu'_{s,xy}(\lambda)$

$\mu_a \ (cm^{-1})$

$\mu_{a,xy}(\lambda)$

**Fig.6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 2762064 A
- WO 2006086085 A2

### Littérature non-brevet citée dans la description

- **SORGATO V.** Non-contact quantitative diffuse reflectance spectroscopy. *Proceedings of SPIE,* 2015, vol. 9538, 95380U
- **NICHOLS B.** Performance of a lookup table-based approach for measuring tissue optical properties with diffuse optical spectroscopy. *Journal of Biomedical Optics,* Mai 2012, vol. 17 (5
- **NICHOLS BS et al.** A Quantitative Diffuse Reflectance Imaging (QDRI) System for Comprehensive Surveillance of the Morphological Landscape in Breast Tumor Margins. *PLoS ONE,* 2015, vol. 10
- **DHAR S.** A diffuse reflectance spectral imaging system for tumor imaging assessment using custom annular photodiode arrays. *Biomedical Optics Express,* 2012, vol. 3 (12
- **BJORGAN A.** Estimation of skin optical parameters for real-time hyperspectral imaging applications. *J. Biomed. Opt.,* 04 Juin 2014, vol. 19 (6), 066003